# EUROPEAN PATENT SPECIFICATION

(11) **EP 3 703 671 B1**
(45) Date of publication and mention of the grant of the patent: **08.01.2025**
(21) Application number: 18872157.5
(22) Date of filing: 30.10.2018
(51) Int. Cl.: A61K 36/185, A61K 31/05, A61K 31/352, A24B 15/167, A61K 9/20

(54) **NEW CANNABIS COMPOSITIONS AND INDUSTRIAL METHODS FOR PRODUCTION THEREOF**
NEUE CANNABISZUSAMMENSETZUNGEN UND INDUSTRIELLE VERFAHREN ZU IHRER HERSTELLUNG
NOUVELLES COMPOSITIONS DE CANNABIS ET PROCÉDÉS INDUSTRIELS POUR LA PRODUCTION DE CES DERNIÈRES

(30) Priority: 31.10.2017 US 201762579834 P; 22.07.2018 US 201862701812 P
(43) Date of publication of application: 09.09.2020
(73) Proprietor: Buzzelet Development And Technologies Ltd, 3065101 Or-Akiva (IL)
(72) Inventor: EYAL, Aharon, 9362921 Jerusalem (IL)
(74) Representative: Harrison IP Limited
(86) International application number: PCT/IB2018/058504
(87) International publication number: WO 2019/087074

(56) References cited:
- WO-A1-2016/092376
- WO-A1-2017/072704
- WO-A1-2017/182950
- WO-A2-2016/001923
- US-A1- 2015 265 720
- US-A1- 2016 074 450
- US-A1- 2017 188 623
- US-A1- 2017 202 895

## Description

### Field of the Invention

The field of art to which this invention generally pertains is cannabis compositions, and specifically new cannabinoid compositions and industrial methods for production thereof.

### Background of the invention

The therapeutic effects of cannabis are known for thousands of years, but only recently the mechanisms of operation are being uncovered.

The main active components are named cannabinoids. So far, more than 100 cannabinoids were discovered. Cannabis plants contain multiple cannabinoids, sometimes dozens of them. The most known cannabinoids are tetrahydrocannabinol (THC) and cannabidiol (CBD). Others include cannabigerol (CBG), cannabichromene (CBC) tetrahydrocannabivarin (THCV) Cannabidivarin (CBDV) and cannabinol (CBN). Cannabinoids are found in various parts of the cannabis plant. Typically, their concentration is greatest in the flower (the bud), particularly in the trichomes.

In the cannabis plant, these cannabinoids are mostly in their acid form. For most therapeutic applications, cannabinoids are heat treated to effect thermal decarboxylation of the cannabinoids, which is sometimes referred to as activation.

Cannabis plants also include other components, e.g. terpenes (terpenoids) and falvenoids. There are claims that terpenes impact the mechanism of operation of the cannabinoids. Known cannabis terpenes include pinene, limonene, linalool, caryophyllene, myrcene, humulene, borneol, eucalyptol, terpineol, nerolidol, phytol, geraniol, bisabolol and camphene.

There are hundreds of cannabis plant strains, which differ in many aspects, including their cannabinoids content. For example, some are high on THC (more than 20wt%), while others have high CBD/THC ratios. There are also major differences in the terpenes content.

Therapeutic cannabis is used in various forms, e.g. smoking, vaporizers, oils, ointments, pills, cookies, drinks, sprays, including nasal ones, etc. In vaporizers, plant material, oil or other forms are heated to a temperature and for a time suitable for decarboxylation and evaporation and the patient inhales the formed vapors.

Therapeutic use requires high standard of manufacture, high product quality, accuracy in dosing and repeatability, so that the patient gets each time the compositions found most suitable for her or him.

Regulations in many countries are changing these days to allow therapeutic use of cannabis and the number of treated patients is already many millions. There is therefore desire for industrial production generating products at best quality standards.

The vast majority of the patients smoke their medical cannabis, which in current practice limits treatment options to the compositions present in grown cannabis strains. This presents a challenge to the treatment. For example, there are many strains with various THC contents and minimal CBD content, as well as strains with high CBD content and minimal THC content, but there is only limited availability of strains with THC to CBD weight/weight ratio of 5/1, 4/1, 3/1, 2/1, 1/1, 1/2, 1/3, 1/4 or 1/5. Various studies have shown that such missing compositions could have been the best ones for treating various indications. Alternatively or additionally, such missing compositions are desired for specific stages of the treatment, e.g. the starting-up phase, or in particular hours of the day. For example, the Israeli regulator has selected 11 medical cannabis compositions to be prescribed and has determined which one to be used at various stages of the treatment. A major fraction of those compositions cannot be supplied by the about 100 strains currently grown in Israel. Development and cultivation of new strains to suit those required compositions might be feasible, but costly and time consuming. New cultivation practices may have to be developed and adopted at additional cost. Additionally, there are compositions that even newly developed strains could probably not reach. Those include compositions where total cannabinoid concentration is high, e.g. higher than 30%.

Hence, there is a strong need for cannabis plant products of those missing compositions that are reliable, accurate and suitable for use by treated patients. There is also a need for method to produce such products on industrial scale and at industrial production rate.

United States Publication No. US 2017/0188623 discloses a method of manufacturing standardized cannabis cigarettes that may comprise drying harvested cannabis plants at room temperature with a humidity of between about 45% and 75%, wherein plant material, including at least one of stems and seeds, may be removed to provide a refined cannabis.

United States Publication No. US 2017/0202895 discloses cannabis pellets and a method of preparation thereof, wherein the method may include providing a cannabis crop, and extracting a cannabinoid resin comprising a tetrahydrocannabinol extract and a cannabidiol extract from the cannabis crop, and formulating the cannabinoid resin into compressed pellets.

Japanese Publication No. JP 2017 530731 discloses a method, apparatus and system for pulmonary delivery of active agents, wherein vaporization is carried out by controllably heating a plant material to vaporize a predetermined vaporization amount of the drug and subjecting the subject to at least one predetermined vaporization amount of pharmacologically active agent in the plant material.

### Summary of the invention

According to an aspect of some embodiments of the present invention, there is provided a homogeneous cannabis plant material composition comprising tetrahydrocannabinol (THC) and cannabidiol (CBD), wherein the plant material is in the form of comminuted particles, and wherein
(i) tetrahydrocannabinol and cannabidiol concentrations are selected from the group consisting of
   (a) 0.01-6wt% tetrahydrocannabinol and 18-24wt% cannabidiol;
   (b) 0.01-6wt% tetrahydrocannabinol and 24-28wt% cannabidiol;
   (c) 0.01-6wt% tetrahydrocannabinol and more than 28wt% cannabidiol;
   (d) 6-12wt% tetrahydrocannabinol and 12-18wt% cannabidiol;
   (e) 6-12wt % tetrahydrocannabinol and 18-24wt% cannabidiol;
   (f) 6-12wt % tetrahydrocannabinol and more than 24wt% cannabidiol;
   (g) 12-18wt% tetrahydrocannabinol and 6-12wt% cannabidiol;
   (h) 12-18wt% tetrahydrocannabinol and 12-18wt% cannabidiol;
   (i) 12-18wt% tetrahydrocannabinol and 18-24wt% cannabidiol;
   (j) 12-18wt % tetrahydrocannabinol and more than 24wt% cannabidiol;
   (k) 18-24wt% tetrahydrocannabinol and 6-12wt% cannabidiol;
   (l) 18-24wt% tetrahydrocannabinol and 12-18wt% cannabidiol;
   (m) 18-24wt% tetrahydrocannabinol and 18-24wt% cannabidiol;
   (n) 18-24wt % tetrahydrocannabinol and more than 24wt% cannabidiol;
   (o) 24-30wt% tetrahydrocannabinol and 6-12wt% cannabidiol;
   (p) 24-30wt% tetrahydrocannabinol and 12-18wt% cannabidiol;
   (q) 30-36wt% tetrahydrocannabinol and 0.01-6wt% cannabidiol;
   (r) 30-36wt% tetrahydrocannabinol and 6-12wt% cannabidiol;
   (s) 30-36wt% tetrahydrocannabinol and 12-18wt% cannabidiol;
   (t) more than 36wt% tetrahydrocannabinol and 0.01-3wt% cannabidiol;
   (u) more than 36wt% tetrahydrocannabinol and 3-6wt% cannabidiol; or
   (v) more than 36wt% tetrahydrocannabinol and more than6wt% cannabidiol; and wherein
(ii) at least 70 wt% of the comminuted cannabis plant material is of a size greater than 0.3 millimeter and less than 4.2millimeters;
(iii) the composition comprises at least 1wt% moisture and less than 20wt% moisture;
(iv) the homogeneous composition, when separated into at least a 10 gram sample, contains a tetrahydrocannabinol concentration in a 1 gram fraction of the at least 10 gram sample within 15% of the tetrahydrocannabinol concentration in a separate 1gram fraction of said at least 10 gram sample; and
(v) and the homogeneous composition, when separated into at least a 10 gram sample, contains a cannabidiol concentration in a 1 gram fraction of the at least 10 gram sample within 15% of the cannabidiol concentration in a separate 1gram fraction of said at least 10 gram sample.

According to an embodiment, the tetrahydrocannabinol comprises tetrahydrocannabinol and tetrahydrocannabinolic acid and wherein the weight per weight ratio between tetrahydrocannabinol and tetrahydrocannabinolic acid is in the range between 20 to 1 and 1 to 20 and wherein the cannabidiol comprises cannabidiol and cannabidiolic acid and wherein the weight per weight ratio between cannabidiol and cannabidiolic acid is in the range between 20 to 1 and 1 to 20.

According to an embodiment, the homogeneous composition has a bulk density greater than 0.02 gram/milliliter and less than 0.6 gram/milliliter.

According to an embodiment, there is provided a product comprising the homogeneous composition as disclosed herein, wherein the product is selected from the group consisting of a cigarette and a vaporizer cartridge.

According to an aspect of some embodiments of the present invention, there is provided a homogeneous tablet, comprising tetrahydrocannabinol (THC) and cannabidiol (CBD), wherein
(i) tetrahydrocannabinol and cannabidiol and cannabidiol concentrations are:
   (a) 0.01-6wt% tetrahydrocannabinol and 18-24wt% cannabidiol;
   (b) 0.01-6wt% tetrahydrocannabinol and 24-28wt% cannabidiol;
   (c) 0.01-6wt% tetrahydrocannabinol and more than 28wt% cannabidiol;
   (d) 6-12wt% tetrahydrocannabinol and 12-18wt% cannabidiol;
   (e) 6-12wt % tetrahydrocannabinol and 18-24wt% cannabidiol;
   (f) 6-12wt % tetrahydrocannabinol and more than 24wt% cannabidiol;
   (g) 12-18wt% tetrahydrocannabinol and 6-12wt% cannabidiol;
   (h) 12-18wt% tetrahydrocannabinol and 12-18wt% cannabidiol;
   (i) 12-18wt% tetrahydrocannabinol and 18-24wt% cannabidiol;
   (j) 12-18wt % tetrahydrocannabinol and more than 24wt% cannabidiol;
   (k) 18-24wt% tetrahydrocannabinol and 6-12wt% cannabidiol;
   (l) 18-24wt% tetrahydrocannabinol and 12-18wt% cannabidiol;
   (m) 18-24wt% tetrahydrocannabinol and 18-24wt% cannabidiol;
   (n) 18-24wt % tetrahydrocannabinol and more than 24wt% cannabidiol;
   (o) 24-30wt% tetrahydrocannabinol and 6-12wt% cannabidiol;
   (p) 24-30wt% tetrahydrocannabinol and 12-18wt% cannabidiol;
   (q) 30-36wt% tetrahydrocannabinol and 0.01-6wt% cannabidiol;
   (r) 30-36wt% tetrahydrocannabinol and 6-12wt% cannabidiol;
   (s) 30-36wt% tetrahydrocannabinol and 12-18wt% cannabidiol;
   (t) More than 36wt% tetrahydrocannabinol and 0.01-3wt% cannabidiol;
   (u) More than 36wt% tetrahydrocannabinol and 3-6wt% cannabidiol; or
   (v) More than 36wt% tetrahydrocannabinol and more than-6wt% cannabidiol;
(ii) said tablet moisture content is greater than 1wt% and less than 20wt% ;
(iii) said tablet bulk density is at least 0.1 gram/milliliter and less than 1.2 gram/milliliter;
(iv) the tetrahydrocannabinol concentration in a fraction of said tablet, is within 15% of the tetrahydrocannabinol concentration in a separate fraction of said tablet;
(v) the cannabidiol concentration in a fraction of said tablet, is within 15% of the cannabidiol concentration in a separate fraction of said tablet; and (vi) said tablet comprises at least 70wt% cannabis plant material.

According to an embodiment, the cannabidiol comprises cannabidiol and cannabinolic acid and wherein the weight per weight ratio between cannabidiol and cannabidiolic acid is in the range between 20 to 1 and wherein the tetrahydrocannabinol comprises tetrahydrocannabinol and tetrahydrocannabinolic acid and 1 to 20 and wherein a weight per weight ratio between tetrahydrocannabinol and tetrahydrocannabinolic acid is in the range between 20 to 1 and 1 to 20.

According to an embodiment, there is provided a method for producing the homogeneous comminuted cannabis plant material composition, as disclosed herein, the method comprising
(i) providing a tetrahydrocannabinol-comprising cannabis plant material;
(ii) providing a cannabidiol-comprising cannabis plant material;
(iii) (a) comminuting said tetrahydrocannabinol-comprising cannabis plant material, and said cannabidiol-comprising cannabis plant material whereby two separate comminuted homogeneous cannabis plant materials are formed, and/or (b) comminuting one of said tetrahydrocannabinol-comprising cannabis plant materials, and said cannabidiol-comprising cannabis plant material, whereby a comminuted homogeneous cannabis plant material and a non-comminuted cannabis plant material are obtained and extracting the non-comminuted cannabis plant material to form a cannabinoid-comprising extract; and
(iv) (a) combining said two separate comminuted homogeneous cannabis plant materials of step (iii)(a) in a proportion calculated to provide said tetrahydrocannabinol and cannabidiol concentrations of said composition, and/or (b) combining said comminuted homogeneous cannabis plant material and said cannabinoid-comprising extract of step (iii)(b) in a proportion to provide said tetrahydrocannabinol and cannabidiol concentrations of said homogeneous comminuted cannabis plant material composition.

According to an embodiment, said extracting comprises
(i) optionally comminuting the non-comminuted cannabis plant material of step (iii)(b), whereby comminuted cannabis plant material is formed;
(ii) contacting said non-comminuted cannabis plant material or said comminuted cannabis plant material with a liquid extractant for at least 1 minute to form cannabinoid-depleted plant material and a cannabinoid-comprising extractant, and
(iii) separating said cannabinoid-depleted plant material from said cannabinoid-comprising extractant to form separated cannabinoid-comprising extractant.

According to an embodiment, the method further comprises comminuting said two comminuted cannabis plant materials or said comminuted cannabis plant material and said cannabinoid-comprising extract with said cannabinoid-depleted plant material in a proportion calculated to form the said composition.

According to an embodiment, the method comprises blending said comminuted cannabis plant material and said cannabinoid-comprising extract with said cannabinoid-depleted plant material in a proportion calculated to form the said composition.

According to an embodiment, the method further comprises adding said homogeneous composition to a cigarettes tube or a cigarette paper, whereby a cigarette is formed.

According to an embodiment, there is provided a cannabis cigarette produced according to the method disclosed herein, wherein said cigarette contains a top fraction and a bottom fraction and the concentration of tetrahydrocannabinol in a 0.1 gram sample from the top fraction of the cigarette is within 15% of tetrahydrocannabinol concentration in a 0.1 gram sample from the bottom fraction of the cigarette and wherein cannabidiol in a 0.1 gram sample from the top fraction of the cigarette is within 15% of cannabidiol concentration in a 0.1 gram sample from the bottom fraction of the cigarette.

According to an embodiment, there is provided a batch of cannabis cigarettes produced according to the method disclosed herein, wherein the content of tetrahydrocannabinol in one third of the batch of the cigarettes is within 15% of the tetrahydrocannabinol content in a separate third of the batch of the cigarettes and wherein the content of cannabidiol in one third of the batch of the cigarettes is within 15% of the cannabidiol content in a separate third of the batch of the cigarettes.

According to an embodiment, the method further comprises pressing said homogeneous comminuted composition to form a homogeneous cannabis tablet.

According to an embodiment, there is provided a homogeneous cannabis tablet produced according to the method disclosed herein, wherein said tablet has a bulk density of at least 0.1 gram/milliliter and less than 1.2 gram/milliliter.

### Detailed description of the invention

The particulars shown herein are by way of example and for purposes of illustrative discussion of the various embodiments of the present invention only and are presented in the cause of providing what is believed to be the most useful and readily understood description of the principles and conceptual aspects of the invention. In this regard, no attempt is made to show details of the invention in more detail than is necessary for a fundamental understanding of the invention, the description making apparent to those skilled in the art how the several forms of the invention may be embodied in practice.

The present invention will now be described by reference to more detailed embodiments. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless indicated otherwise, percent is weight percent (%wt).

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Additional advantages of the invention will be set forth in part in the description which follows, and in part will be obvious from the description, or may be learned by practice of the invention. It is to be understood that both the foregoing general description and the following detailed description are exemplary and explanatory only and are not restrictive of the invention, as claimed.

Provided is a homogeneous comminuted cannabis plant material composition is described comprising tetrahydrocannabinol (THC) and cannabidiol (CBD), wherein
(i) tetrahydrocannabinol and cannabidiol concentrations are
   (a) 0.01-6weight(wt)% tetrahydrocannabinol and 0.01-6wt% cannabidiol;
   (b) 0.01-6wt% tetrahydrocannabinol and 6-12wt% cannabidiol;
   (c) 0.01-6wt% tetrahydrocannabinol and 12-18wt% cannabidiol;
   (d) 0.01-6wt% tetrahydrocannabinol and 18-24wt% cannabidiol;
   (e) 0.01-6wt% tetrahydrocannabinol and 24-28wt% cannabidiol;
   (f) 0.01-6wt% tetrahydrocannabinol and more than 28wt% cannabidiol;
   (g) 6-12wt% tetrahydrocannabinol and 0.01-6wt% cannabidiol;
   (h) 6-12wt% tetrahydrocannabinol and 6-12wt% cannabidiol;
   (i) 6-12wt% tetrahydrocannabinol and 12-18wt% cannabidiol;
   (j) 6-12wt % tetrahydrocannabinol and 18-24wt% cannabidiol;
   (k) 6-12wt % tetrahydrocannabinol and more than 24wt% cannabidiol;
   (l) 12-18wt% tetrahydrocannabinol and 0.01-6wt% cannabidiol;
   (m) 12-18wt% tetrahydrocannabinol and 6-12wt% cannabidiol;
   (n) 12-18wt% tetrahydrocannabinol and 12-18wt% cannabidiol;
   (o) 12-18wt% tetrahydrocannabinol and 18-24wt% cannabidiol;
   (p) 12-18wt % tetrahydrocannabinol and more than 24wt% cannabidiol;
   (q) 18-24wt% tetrahydrocannabinol and 0.01-6wt% cannabidiol;
   (r) 18-24wt% tetrahydrocannabinol and 6-12wt% cannabidiol;
   (s) 18-24wt% tetrahydrocannabinol and 12-18wt% cannabidiol;
   (t) 18-24wt% tetrahydrocannabinol and 18-24wt% cannabidiol;
   (u) 18-24wt % tetrahydrocannabinol and more than 24wt% cannabidiol;
   (v) 24-30wt% tetrahydrocannabinol and 0.01-6wt% cannabidiol;
   (w) 24-30wt% tetrahydrocannabinol and 6-12wt% cannabidiol;
   (x) 24-30wt% tetrahydrocannabinol and 12-18wt% cannabidiol;
   (y) 30-36wt% tetrahydrocannabinol and 0.01-6wt% cannabidiol;
   (z) 30-36wt% tetrahydrocannabinol and 6-12wt% cannabidiol;
   (aa) 30-36wt% tetrahydrocannabinol and 12-18wt% cannabidiol;
   (bb)more than 36wt% tetrahydrocannabinol and 0.01-3wt% cannabidiol;
   (cc) more than 36wt% tetrahydrocannabinol and 3-6wt% cannabidiol; or
   (dd) more than 36wt% tetrahydrocannabinol and more than 6wt% cannabidiol;
(ii) at least 70 wt% of the comminuted cannabis plant material is of a size greater than 0.3 millimeter and less than 4.2 millimeters;
(iii) the composition comprises at least 1wt% moisture and less than 20wt% moisture;
(iv) the homogeneous composition, when separated into at least a 10 gram sample, contains a tetrahydrocannabinol concentration in a 1 gram fraction of the at least 10 gram sample within 15% of the tetrahydrocannabinol concentration in a separate 1gram fraction of said at least 10 gram sample; and
(v) the homogeneous composition, when separated into at least a 10 gram sample, contains a cannabidiol concentration in a 1 gram fraction of the at least 10 gram sample within 15% of the cannabidiol concentration in a separate 1gram fraction of said at least 10 gram sample.

According to an embodiment, said homogeneous comminuted cannabis plant material composition comprises 0.01-6wt% tetrahydrocannabinol and 0.01-6wt% cannabidiol.

According to an embodiment, said homogeneous composition comprises at least 0.01wt% tetrahydrocannabinol, at least 2wt% or at least 4wt%. According to an embodiment, said homogeneous composition comprises less than 6wt% tetrahydrocannabinol, less than 4wt% or less than 2wt%. According to an embodiment, said homogeneous composition comprises at least 0.1wt% cannabidiol, at least 2wt% or at least 4wt%.

According to an embodiment, said homogeneous composition comprises less than 6wt% cannabidiol, less than 4wt% or less than 2wt%.

According to an embodiment, said homogeneous comminuted cannabis plant material composition comprises 0.01-6wt% tetrahydrocannabinol and 6-12wt% cannabidiol.

According to an embodiment, said homogeneous composition comprises at least 0.01wt% tetrahydrocannabinol, at least 2wt% or at least 4wt%.

According to an embodiment, said homogeneous composition comprises less than 6wt% tetrahydrocannabinol, less than 4wt% or less than 2wt%. According to an embodiment, said homogeneous composition comprises at least 6wt% cannabidiol, at least 8wt% or at least 10wt%.

According to an embodiment, said homogeneous composition comprises less than 12wt% cannabidiol, less than 10wt% or less than 8wt%.

According to an embodiment, said homogeneous comminuted cannabis plant material composition comprises 0.01-6wt% tetrahydrocannabinol and 12-18wt% cannabidiol.

According to an embodiment, said homogeneous composition comprises at least 0.01wt% tetrahydrocannabinol, at least 2wt% or at least 4wt%.

According to an embodiment, said homogeneous composition comprises less than 6wt% tetrahydrocannabinol, less than 4wt% or less than 2wt%.

According to an embodiment, said homogeneous composition comprises at least 12wt% cannabidiol, at least 14wt% or at least 16wt%.

According to an embodiment, said homogeneous composition comprises less than 18wt% cannabidiol, less than 16wt% or less than 14wt%.

According to an embodiment, said homogeneous comminuted cannabis plant material composition comprises 0.01-6wt% tetrahydrocannabinol and 18-24wt% cannabidiol.

According to an embodiment, said homogeneous composition comprises at least 0.01wt% tetrahydrocannabinol, at least 2wt% or at least 4wt%.

According to an embodiment, said homogeneous composition comprises less than 6wt% tetrahydrocannabinol, less than 4wt% or less than 2wt%.

According to an embodiment, said homogeneous composition comprises at least 18wt% cannabidiol, at least 20wt% or at least 22wt%.

According to an embodiment, said homogeneous composition comprises less than 24wt% cannabidiol, less than 22wt% or less than 20wt%.

According to an embodiment, said homogeneous comminuted cannabis plant material composition comprises 0.01-6wt% tetrahydrocannabinol and more than 24wt% cannabidiol.

According to an embodiment, said homogeneous composition comprises at least 0.01wt% tetrahydrocannabinol, at least 2wt% or at least 4wt%.

According to an embodiment, said homogeneous composition comprises less than 6wt% tetrahydrocannabinol, less than 4wt% or less than 2wt%.

According to an embodiment, said homogeneous composition comprises at least 24wt% cannabidiol, at least 26wt%, at least28wt% or at least 30wt%.

According to an embodiment, said homogeneous comminuted cannabis plant material composition comprises 6-12wt% tetrahydrocannabinol and 0.01-6wt% cannabidiol.

According to an embodiment, said homogeneous composition comprises at least 6wt% tetrahydrocannabinol, at least 8wt% or at least 10wt%.

According to an embodiment, said homogeneous composition comprises less than 12wt% tetrahydrocannabinol, less than 10wt% or less than 8wt%.

According to an embodiment, said homogeneous composition comprises at least 0.01wt% cannabidiol, at least 2wt% or at least 4wt%.

According to an embodiment, said homogeneous composition comprises less than 6wt% cannabidiol, less than 4wt% or less than 2wt%.

According to an embodiment, said homogeneous comminuted cannabis plant material composition comprises 6-12wt% tetrahydrocannabinol and 6-12wt% cannabidiol.

According to an embodiment, said homogeneous composition comprises at least 6wt% tetrahydrocannabinol, at least 8wt% or at least 10wt%.

According to an embodiment, said homogeneous composition comprises less than 12wt% tetrahydrocannabinol, less than 10wt% or less than 8wt%.

According to an embodiment, said homogeneous composition comprises at least 6wt% cannabidiol, at least 8wt% or at least 10wt%.

According to an embodiment, said homogeneous composition comprises less than 12wt% cannabidiol, less than 10wt% or less than 8wt%.

According to an embodiment, said homogeneous comminuted cannabis plant material composition comprises 6-12wt% tetrahydrocannabinol and 12-18wt% cannabidiol.

According to an embodiment, said homogeneous composition comprises at least 6wt% tetrahydrocannabinol, at least 8wt% or at least 10wt%.

According to an embodiment, said homogeneous composition comprises less than 12wt% tetrahydrocannabinol, less than 10wt% or less than 8wt%.

According to an embodiment, said homogeneous composition comprises at least 12wt% cannabidiol, at least 14wt% or at least 16wt%.

According to an embodiment, said homogeneous composition comprises less than 18wt% cannabidiol, less than 16wt% or less than 14wt%.

According to an embodiment, said homogeneous comminuted cannabis plant material composition comprises 6-12wt % tetrahydrocannabinol and 18-24wt% cannabidiol.

According to an embodiment, said homogeneous composition comprises at least 6wt% tetrahydrocannabinol, at least 8wt% or at least 10wt%.

According to an embodiment, said homogeneous composition comprises less than 12wt% tetrahydrocannabinol, less than 10wt% or less than 8wt%. According to an embodiment, said homogeneous composition comprises at least 18wt% cannabidiol, at least 20wt% or at least 22wt%.

According to an embodiment, said homogeneous composition comprises less than 24wt% cannabidiol, less than 22wt% or less than 20wt%.

According to an embodiment, said homogeneous comminuted cannabis plant material composition comprises 6-12wt % tetrahydrocannabinol and more than 24wt% cannabidiol.

According to an embodiment, said homogeneous composition comprises at least 6wt% tetrahydrocannabinol, at least 8wt% or at least 10wt%. According to an embodiment, said homogeneous composition comprises less than 12wt% tetrahydrocannabinol, less than 10wt% or less than 8wt%.

According to an embodiment, said homogeneous composition comprises at least 24wt% cannabidiol, at least 26wt%, at least28wt% or at least 30wt%.

According to an embodiment, said homogeneous comminuted cannabis plant material composition comprises 12-18wt% tetrahydrocannabinol and 0.01-6wt% cannabidiol. According to an embodiment, said homogeneous composition comprises at least 12wt% tetrahydrocannabinol, at least 14wt% or at least 16wt%. According to an embodiment, said homogeneous composition comprises less than 18wt% tetrahydrocannabinol, less than 16wt% or less than 14wt%. According to an embodiment, said homogeneous composition comprises at least 0.1wt% cannabidiol, at least 2wt% or at least 4wt%. According to an embodiment, said homogeneous composition comprises less than 6wt% cannabidiol, less than 4wt% or less than 2wt%.

According to an embodiment, said homogeneous comminuted cannabis plant material composition comprises 12-18wt% tetrahydrocannabinol and 6-12wt% cannabidiol. According to an embodiment, said homogeneous composition comprises at least 12wt% tetrahydrocannabinol, at least 14wt% or at least 16wt%. According to an embodiment, said homogeneous composition comprises less than 18wt% tetrahydrocannabinol, less than 16wt% or less than 14wt%. According to an embodiment, said homogeneous composition comprises at least 6wt% cannabidiol, at least 8wt% or at least 10wt%. According to an embodiment, said homogeneous composition comprises less than 12wt% cannabidiol, less than 10wt% or less than 8wt%.

According to an embodiment, said homogeneous comminuted cannabis plant material composition comprises12-18wt% tetrahydrocannabinol and 12-18wt% cannabidiol. According to an embodiment, said homogeneous composition comprises at least 12wt% tetrahydrocannabinol, at least 14wt% or at least 16wt%. According to an embodiment, said homogeneous composition comprises less than 18wt% tetrahydrocannabinol, less than 16wt% or less than 14wt%. According to an embodiment, said homogeneous composition comprises at least 12wt% cannabidiol, at least 14wt% or at least 16wt%. According to an embodiment, said homogeneous composition comprises less than 18wt% cannabidiol, less than 16wt% or less than 14wt%.

According to an embodiment, said homogeneous comminuted cannabis plant material composition comprises 12-18wt% tetrahydrocannabinol and 18-24wt% cannabidiol. According to an embodiment, said homogeneous composition comprises at least 12wt% tetrahydrocannabinol, at least 14wt% or at least 16wt%. According to an embodiment, said homogeneous composition comprises less than 18wt% tetrahydrocannabinol, less than 16wt% or less than 14wt%. According to an embodiment, said homogeneous composition comprises at least 18wt% cannabidiol, at least 20wt% or at least 22wt%. According to an embodiment, said homogeneous composition comprises less than 24wt% cannabidiol, less than 22wt% or less than 20wt%.

According to an embodiment, said homogeneous comminuted cannabis plant material composition comprises 12-18wt % tetrahydrocannabinol and more than 24wt% cannabidiol. According to an embodiment, said homogeneous composition comprises at least 12wt% tetrahydrocannabinol, at least 14wt% or at least 16wt%. According to an embodiment, said homogeneous composition comprises less than 18wt% tetrahydrocannabinol, less than 16wt% or less than 14wt%. According to an embodiment, said homogeneous composition comprises at least 24wt% cannabidiol, at least 26wt%, at least28wt% or at least 30wt%.

According to an embodiment, said homogeneous comminuted cannabis plant material composition comprises 18-24wt% tetrahydrocannabinol and 0.01-6wt% cannabidiol. According to an embodiment, said homogeneous composition comprises at least 18wt% tetrahydrocannabinol, at least 20wt% or at least 22wt%. According to an embodiment, said homogeneous composition comprises less than 24wt% tetrahydrocannabinol, less than 22wt% or less than 20wt%. According to an embodiment, said homogeneous composition comprises at least 0.1wt% cannabidiol, at least 2wt% or at least 4wt%. According to an embodiment, said homogeneous composition comprises less than 6wt% cannabidiol, less than 4wt% or less than 2wt%.

According to an embodiment, said homogeneous comminuted cannabis plant material composition comprises 18-24wt% tetrahydrocannabinol and 6-12wt% cannabidiol. According to an embodiment, said homogeneous composition comprises at least 18wt% tetrahydrocannabinol, at least 20wt% or at least 22wt%. According to an embodiment, said homogeneous composition comprises less than 24wt% tetrahydrocannabinol, less than 22wt% or less than 20wt%. According to an embodiment, said homogeneous composition comprises at least 6wt% cannabidiol, at least 8wt% or at least 10wt%. According to an embodiment, said homogeneous composition comprises less than 12wt% cannabidiol, less than 10wt% or less than 8wt%.

According to an embodiment, said homogeneous comminuted cannabis plant material composition comprises 18-24wt% tetrahydrocannabinol and 12-18wt% cannabidiol. According to an embodiment, said homogeneous composition comprises at least 18wt% tetrahydrocannabinol, at least 20wt% or at least 22wt%. According to an embodiment, said homogeneous composition comprises less than 24wt% tetrahydrocannabinol, less than 22wt% or less than 20wt%. According to an embodiment, said homogeneous composition comprises at least 12wt% cannabidiol, at least 14wt% or at least 16wt%. According to an embodiment, said homogeneous composition comprises less than 18wt% cannabidiol, less than 16wt% or less than 14wt%.

According to an embodiment, said homogeneous comminuted cannabis plant material composition comprises 18-24wt% tetrahydrocannabinol and 18-24wt% cannabidiol. According to an embodiment, said homogeneous composition comprises at least 18wt% tetrahydrocannabinol, at least 20wt% or at least 22wt%. According to an embodiment, said homogeneous composition comprises less than 24wt% tetrahydrocannabinol, less than 22wt% or less than 20wt%. According to an embodiment, said homogeneous composition comprises at least 18wt% cannabidiol, at least 20wt% or at least 22wt%. According to an embodiment, said homogeneous composition comprises less than 24wt% cannabidiol, less than 22wt% or less than 20wt%. According to an embodiment, said homogeneous comminuted cannabis plant material composition comprises 18-24wt % tetrahydrocannabinol and more than 24wt% cannabidiol.

According to an embodiment, said homogeneous composition comprises at least 18wt% tetrahydrocannabinol, at least 20wt% or at least 22wt%. According to an embodiment, said homogeneous composition comprises less than 24wt% tetrahydrocannabinol, less than 22wt% or less than 20wt%. According to an embodiment, said homogeneous composition comprises at least 24wt% cannabidiol, at least 26wt%, at least28wt% or at least 30wt%.

According to an embodiment, said homogeneous comminuted cannabis plant material composition comprises more than 24wt% tetrahydrocannabinol and 0.01-6wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 0.1wt% cannabidiol, at least 2wt% or at least 4wt%. According to an embodiment, said homogeneous composition comprises less than 6wt% cannabidiol, less than 4wt% or less than 2wt%.

According to an embodiment, said homogeneous comminuted cannabis plant material composition comprises more than 24wt% tetrahydrocannabinol and 6-12wt% cannabidiol. According to an embodiment, said homogeneous composition comprises at least 24wt% tetrahydrocannabinol, at least 26wt%, at least 28wt% or at least 30wt%. According to an embodiment, said homogeneous composition comprises at least 6wt% cannabidiol, at least 8wt% or at least 10wt%. According to an embodiment, said homogeneous composition comprises less than 12wt% cannabidiol, less than 10wt% or less than 8wt%.

According to an embodiment, said homogeneous comminuted cannabis plant material composition comprises more than 24wt% tetrahydrocannabinol and 12-18wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 12 wt% cannabidiol, at least 14wt% or at least 16wt%. According to an embodiment, said homogeneous composition comprises less than 18wt% cannabidiol, less than 16wt% or less than 14wt%.

According to an embodiment, said homogeneous comminuted cannabis plant material composition comprises more than 24wt% tetrahydrocannabinol and 18-24wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 18wt% cannabidiol, at least 20wt% or at least 22wt%. According to an embodiment, said homogeneous composition comprises less than 24wt% cannabidiol, less than 22wt% or less than 20wt%.

According to an embodiment, said homogeneous comminuted cannabis plant material composition comprises more than 24wt% tetrahydrocannabinol and more than 24wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 24wt% cannabidiol, at least 26wt%, at least28wt% or at least 30wt%.

According to an embodiment, said homogeneous comminuted cannabis plant material composition comprises 24-30wt% tetrahydrocannabinol and 0.01-6wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 0.01wt% cannabidiol, at least 2wt%, at least4wt% or at least 6wt%.

According to an embodiment, said homogeneous comminuted cannabis plant material composition comprises 24-30wt% tetrahydrocannabinol and 6-12wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 6wt% cannabidiol, at least 8wt%, at least 10wt% or at least 12wt%.

According to an embodiment, said homogeneous comminuted cannabis plant material composition comprises 24-30wt% tetrahydrocannabinol and 12-18wt% cannabidiol. According to an embodiment, said homogeneous composition comprises at least 24wt% tetrahydrocannabinol, at least 26wt%, at least28wt% or at least 30wt%. According to an embodiment, said homogeneous composition comprises at least 12wt% cannabidiol, at least 14wt%, at least 16wt% or at least 18wt%.

According to an embodiment, said homogeneous comminuted cannabis plant material composition comprises 30-36wt% tetrahydrocannabinol and 0.01-6wt% cannabidiol. According to an embodiment, said homogeneous composition comprises at least 30wt% tetrahydrocannabinol, at least 32wt%, at least 34wt% or at least 36wt%. According to an embodiment, said homogeneous composition comprises at least 0.01wt% cannabidiol, at least 2wt%, at least4wt% or at least 6wt%.

According to an embodiment, said homogeneous comminuted cannabis plant material composition comprises 30-36wt% tetrahydrocannabinol and 6-12wt% cannabidiol. According to an embodiment, said homogeneous composition comprises at least 30wt% tetrahydrocannabinol, at least 32wt%, at least 34wt% or at least 36wt%. According to an embodiment, said homogeneous composition comprises at least 6wt% cannabidiol, at least 8wt%, at least 10wt% or at least 12wt%.

According to an embodiment, said homogeneous comminuted cannabis plant material composition comprises 30-36wt% tetrahydrocannabinol and 12-18wt% cannabidiol. According to an embodiment, said homogeneous composition comprises at least 30wt% tetrahydrocannabinol, at least 32wt%, at least 34wt% or at least 36wt%. According to an embodiment, said homogeneous composition comprises at least 12wt% cannabidiol, at least 14wt%, at least 16wt% or at least 18wt%.

According to an embodiment, said homogeneous comminuted cannabis plant material composition comprises more than 36wt% tetrahydrocannabinol and 0.01-6wt% cannabidiol. According to an embodiment, said homogeneous composition comprises at least 0.01wt% cannabidiol, at least 2wt%, at least4wt% or at least 6wt%.

According to an embodiment, said homogeneous comminuted cannabis plant material composition comprises more than 36wt% tetrahydrocannabinol and 6-12wt% cannabidiol. According to an embodiment, said homogeneous composition comprises at least 6wt% cannabidiol, at least 8wt%, at least 10wt% or at least 12wt%.

According to an embodiment, said homogeneous comminuted cannabis plant material composition comprises more than 36wt% tetrahydrocannabinol and 12-18wt% cannabidiol.

According to an embodiment, said homogeneous composition comprises at least 12wt% cannabidiol, at least 14wt%, at least 16wt% or at least 18wt%.

According to an embodiment, said homogeneous comminuted cannabis plant material composition comprises more than 36wt% tetrahydrocannabinol and more then 6wt% cannabidiol.

As used herein, the term cannabis plant material refers to material resulting from cannabis plant, e.g. fractions of cannabis plant as such, and products of processing fractions of cannabis plant, e.g. drying and curing. According to an embodiment at least 70wt% of said composition comprises cannabis plant material, at least 75wt%, least 80wt%, least 85wt%, least 90wt% or at least 95wt%.

According to an embodiment at least 70wt% of the cannabis plant material in said composition is derived from cannabis flower (also referred to as cannabis bud), at least 75wt%, least 80wt%, least 85wt%, least 90wt% or at least 95wt%. As used herein, derived from cannabis flower refers to being cannabis flower or being a product or processing cannabis flower, e.g. drying and/or comminuting a cannabis flower. According to an embodiment, at least a fraction of said cannabis plant material in said composition comprises dried cannabis plant material, cured cannabis plant material or dried and cured cannabis plant material.

According to an embodiment said composition comprises comminuted cannabis plant material. As used herein the term comminuted material refers to a collection of small particles. According to an embodiment at least 70wt% of the particles, at least 80wt%, at least 90wt% or at least 95wt%, are of a size greater than 0.3 millimeter (mm), greater than 0.5mm, greater than 0.7mm, greater than 0.9mm, greater than 1.1mm or greater than 1.3mm. As used herein, particle size refers to the largest dimension of said particles. According to an embodiment at least 70wt% of the particles, at least 80wt%, at least 90wt% or at least 95wt% are of a size less than 4.2mm, less than 4.0mm, less than 3.8mm, less than 3.6mm less than 3.4mm, less than 3.2mm, less than 3.0mm, less than 2.6mm or less than 2.4.

According to an embodiment, said homogeneous comminuted cannabis plant material composition comprises the cannabinoids tetrahydrocannabinol (THC) and cannabidiol (CBD). According to an embodiment, said homogeneous composition comprises at least three cannabinoids, at least four, at least five or at least six. As known in the art, cannabinoids have an acid form and a non-acid form (which is also referred to as decarboxylated form, since it can be generated by decarboxylating the acid form). The acid form is indicated herein by the letter (a) at the end of the cannabinoid acronym, e.g. tetrahydrocannabinolic acid is indicated as THCa, while the decarboxylated form is THC. As used herein, unless specified otherwise, the term cannabinoid and the name of the cannabinoid refers to both the acid form and the decarboxylated form. For example, tetrahydrocannabinol refers to THC and/or THCa and cannabidiol refers to CBDa and/or CBD. According to an embodiment, said homogeneous composition further comprises cannabigerol, cannabichromene tetrahydrocannabivarin, cannabidivarin and cannabinol and combinations thereof. According to an embodiment, said homogeneous composition of Claim 1, comprises less than 1wt% cannabinol, less than 0.8wt%, less than 0.7wt%, less than 0.6wt%, less than 0.5wt% or less than 0.1wt%.

According to an embodiment, said homogeneous composition comprises more than 36 wt% tetrahydrocannabinol and 0.01-9wt% cannabidiol. According to an embodiment, said homogeneous composition comprises at least 36wt% tetrahydrocannabinol. According to an embodiment, said homogeneous composition comprises at least 0.01wt% cannabidiol, at least 3wt%, at least 5% or at least 9wt%. According to an embodiment, said homogeneous composition comprises less than 9wt% cannabidiol, less than 5wt% or less than 3wt%.

According to an embodiment, said homogeneous composition comprises 30-36wt% tetrahydrocannabinol and 0.01-15wt% cannabidiol. According to an embodiment, said homogeneous composition comprises at least 32wt% tetrahydrocannabinol, at least 34wt% or at least 36wt%. According to an embodiment, said homogeneous composition comprises less than 36wt% tetrahydrocannabinol, less than 34wt% or less than 32wt%. According to an embodiment, said homogeneous composition comprises at least 0.01wt% cannabidiol, at least 7wt% or at least 15wt%. According to an embodiment, said homogeneous composition comprises less than 15wt% cannabidiol, less than 10wt% or less than 3wt%.

According to an embodiment, said homogeneous composition comprises 24-30wt% tetrahydrocannabinol and 0.01-18wt% cannabidiol. According to an embodiment, said homogeneous composition comprises at least 26wt% tetrahydrocannabinol, at least 28wt% or at least 30wt%. According to an embodiment, said homogeneous composition comprises less than 30wt% tetrahydrocannabinol, less than 28wt% or less than 26wt%. According to an embodiment, said homogeneous composition comprises at least 0.01wt% cannabidiol, at least 10wt% or at least 16wt%. According to an embodiment, said homogeneous composition comprises less than 18wt% cannabidiol, less than 10wt% or less than 3wt%.

According to an embodiment, said homogeneous composition comprises 18-24wt% tetrahydrocannabinol and 0.01-18wt% cannabidiol. According to an embodiment, said homogeneous composition comprises at least 20wt% tetrahydrocannabinol, at least 22wt% or at least 24wt%. According to an embodiment, said homogeneous composition comprises less than 26wt% tetrahydrocannabinol, less than 24wt% or less than 22wt%. According to an embodiment, said homogeneous composition comprises at least 0.01wt% cannabidiol, at least 10wt% or at least 16wt%. According to an embodiment, said homogeneous composition comprises less than 18wt% cannabidiol, less than 10wt% or less than 3wt%.

According to an embodiment, said homogeneous composition comprises 12-18wt% tetrahydrocannabinol and 0.01-21wt% cannabidiol. According to an embodiment, said homogeneous composition comprises at least 12wt% tetrahydrocannabinol, at least 14wt% or at least 16wt%. According to an embodiment, said homogeneous composition comprises less than 18wt% tetrahydrocannabinol, less than 16wt% or less than 14wt%. According to an embodiment, said homogeneous composition comprises at least 0.01wt% cannabidiol, at least 10wt% or at least 20wt%. According to an embodiment, said homogeneous composition comprises less than 21wt% cannabidiol, less than 10wt% or less than 3wt%.

According to an embodiment, said homogeneous composition comprises 6-12wt% tetrahydrocannabinol and 0.01-24wt% cannabidiol. According to an embodiment, said homogeneous composition comprises at least 6wt% tetrahydrocannabinol, at least 8wt% or at least 10wt%. According to an embodiment, said homogeneous composition comprises less than 12wt% tetrahydrocannabinol, less than 10wt% or less than 8wt%. According to an embodiment, said homogeneous composition comprises at least 0.01wt% cannabidiol, at least 10wt% or at least 22wt%. According to an embodiment, said homogeneous composition comprises less than 24wt% cannabidiol, less than 10wt% or less than 3wt%.

According to an embodiment, said homogeneous composition comprises 0.01-6wt% tetrahydrocannabinol and 0.01-34wt% cannabidiol. According to an embodiment, said homogeneous composition comprises at least 0.01wt% tetrahydrocannabinol, at least 2wt% or at least 4wt%. According to an embodiment, said homogeneous composition comprises less than 6wt% tetrahydrocannabinol, less than 4wt% or less than 2wt%. According to an embodiment, said homogeneous composition comprises at least 0.01wt% cannabidiol, at least 15wt% or at least 30wt%. According to an embodiment, said homogeneous composition comprises less than 34wt% cannabidiol, less than 20wt% or less than 10wt%.

According to an embodiment, said homogeneous composition comprises 16-24wt% tetrahydrocannabinol and 1-7wt% cannabidiol. According to an embodiment, said homogeneous composition comprises at least 16wt% tetrahydrocannabinol, at least 18wt% or at least 20wt%. According to an embodiment, said homogeneous composition comprises less than 24wt% tetrahydrocannabinol, less than 22wt% or less than 20wt%. According to an embodiment, said homogeneous composition comprises at least 1wt% cannabidiol, at least 3wt% or at least 5wt%. According to an embodiment, said homogeneous composition comprises less than 7wt% cannabidiol, less than 5wt% or less than 3wt%.

According to an embodiment, said homogeneous composition comprises 11-18wt% tetrahydrocannabinol and 0.5-5.5wt% cannabidiol. According to an embodiment, said homogeneous composition comprises at least 11wt% tetrahydrocannabinol, at least 13wt% or at least 15wt%. According to an embodiment, said homogeneous composition comprises less than 19wt% tetrahydrocannabinol, less than 17wt% or less than 15wt%. According to an embodiment, said homogeneous composition comprises at least 0.5wt% cannabidiol, at least 1.5wt% or at least 2.5wt%. According to an embodiment, said homogeneous composition comprises less than 5.5wt% cannabidiol, less than 4.5wt% or less than 3.5wt%.

According to an embodiment, said homogeneous composition comprises 6-14wt% tetrahydrocannabinol and 0.2-3.8wt% cannabidiol. According to an embodiment, said homogeneous composition comprises at least 6wt% tetrahydrocannabinol, at least 8wt% or at least 10wt%. According to an embodiment, said homogeneous composition comprises less than 14wt% tetrahydrocannabinol, less than 12wt% or less than 10wt%. According to an embodiment, said homogeneous composition comprises at least 0.5wt% cannabidiol, at least 1wt% or at least 1.5wt%. According to an embodiment, said homogeneous composition comprises less than 3.5wt% cannabidiol, less than 2.5wt% or less than 1.5wt%.

According to an embodiment, said homogeneous composition comprises 6-14wt% tetrahydrocannabinol and 6-14wt% cannabidiol. According to an embodiment, said homogeneous composition comprises at least 6wt% tetrahydrocannabinol, at least 8wt% or at least 10wt%. According to an embodiment, said homogeneous composition comprises less than 14wt% tetrahydrocannabinol, less than 12wt% or less than 10wt%. According to an embodiment, said homogeneous composition comprises at least 6wt% cannabidiol, at least 8wt% or at least 10wt%. According to an embodiment, said homogeneous composition comprises less than 14wt% cannabidiol, less than 12wt% or less than 10wt%.

According to an embodiment, said homogeneous composition comprises 2.5-7.5wt% tetrahydrocannabinol and 6-14wt% cannabidiol. According to an embodiment, said homogeneous composition comprises at least 2.5wt% tetrahydrocannabinol, at least 3.5wt% or at least 4.5wt%. According to an embodiment, said homogeneous composition comprises less than 7.5wt% tetrahydrocannabinol, less than 6.5wt% or less than 5.5wt%. According to an embodiment, said homogeneous composition comprises at least 6wt% cannabidiol, at least 8wt% or at least 10wt%. According to an embodiment, said homogeneous composition comprises less than 14wt% cannabidiol, less than 12wt% or less than 10wt%.

According to an embodiment, said homogeneous composition comprises 0.5-5.5wt% tetrahydrocannabinol and 11-19wt% cannabidiol. According to an embodiment, said homogeneous composition comprises at least 0.5wt% tetrahydrocannabinol, at least 1.5wt% or at least 2.5wt%. According to an embodiment, said homogeneous composition comprises less than 5.5wt% tetrahydrocannabinol, less than 4.5wt% or less than 3.5wt%. According to an embodiment, said homogeneous composition comprises at least 11wt% cannabidiol, at least 13wt% or at least 15wt%. According to an embodiment, said homogeneous composition comprises less than 19wt% cannabidiol, less than 17wt% or less than 15wt%.

According to an embodiment, said homogeneous composition comprises 0.1-2.5wt% tetrahydrocannabinol and 16-24wt% cannabidiol. According to an embodiment, said homogeneous composition comprises at least 0.5wt% tetrahydrocannabinol, at least 1wt% or at least 1.5wt%. According to an embodiment, said homogeneous composition comprises less than 2.5wt% tetrahydrocannabinol, less than 2wt% or less than 1.5wt%. According to an embodiment, said homogeneous composition comprises at least 16wt% cannabidiol, at least 18wt% or at least 20wt%. According to an embodiment, said homogeneous composition comprises less than 24wt% cannabidiol, less than 22wt% or less than 20wt%.

According to an embodiment, said homogeneous composition comprises 0.1-0.5wt% tetrahydrocannabinol and 20-28wt% cannabidiol. According to an embodiment, said homogeneous composition comprises at least 20wt% cannabidiol, at least 22wt% or at least 24wt%. According to an embodiment, said homogeneous composition comprises less than 28wt% cannabidiol, less than 26wt% or less than 24wt%.

According to an embodiment, the tetrahydrocannabinol comprises tetrahydrocannabinol and tetrahydrocannabinolic acid and the weight per weight ratio between tetrahydrocannabinol and tetrahydrocannabinolic acid in said composition is in the range between 20 to 1 and 1 to 20. According to an embodiment, the weight per weight ratio between tetrahydrocannabinol and tetrahydrocannabinolic acid in said composition is less than 18:1, less than 16:1, less than 14:1, less than 12:1, less than 10:1, less than 8:1, less than 6:1, less than 4:1, less than 2:1 or less than 1:1. According to an embodiment, the weight per weight ratio between tetrahydrocannabinol and tetrahydrocannabinolic acid in said composition is greater than 1:20, greater than 1:18, greater than 1:16, greater than 1:14, greater than 1:12, greater than 1:10, greater than 1:8, greater than 1:6, greater than 1:4, greater than 1:2, or greater than 1:1.

According to an embodiment, wherein the cannabinol comprises cannabinol and cannabinolic acid and the weight per weight ratio between cannabidiol and cannabidiolic acid in said composition is in the range between 20 to 1 and 1 to 20. According to an embodiment, the weight per weight ratio between cannabidiol and cannabidiolic acid in said composition is less than 18:1, less than 16:1, less than 14:1, less than 12:1, less than 10:1, less than 8:1, less than 6:1, less than 4:1, less than 2:1 or less than 1:1. According to an embodiment, the weight per weight ratio between cannabidiol and cannabidiolic acid in said composition is greater than 1:20, greater than 1:18, greater than 1:16, greater than 1:14, greater than 1:12, greater than 1:10, greater than 1:8, greater than 1:6, greater than 1:4, greater than 1:2, or greater than 1:1.

According to an embodiment said homogeneous composition further comprises at least one terpene, at least two, at least three, at least four or at least five terpenes. The term "terpene", as used herein, refers to terpenes and/or terpenoids. According to an embodiment, said terpene is selected from the group consisting of pinene, limonene, linalool, caryophyllene, myrcene, humulene, borneol, eucalyptol, terpineol, nerolidol, phytol, geraniol, bisabolol, camphene, beta-amyrin, thujone, citronellol, pulegone, cycloartenol, isomers thereof and combinations thereof.

According to an embodiment said composition comprises moisture. As used herein, the term moisture refers to water contained in the plant material or wetting it. According to an embodiment, moisture content can be determined by loss on drying. According to an embodiment said composition comprises at least 1wt% moisture; at least 3wt%, at least 5wt%, at least 7wt%, at least 9wt%, or at least 11wt%. According to an embodiment said composition comprises less than 20wt% moisture, less than 18wt%, less than 16wt%, or less than 14wt%. According to an embodiment said composition comprises between 10wt% and 14wt% moisture or between 11wt% and 13wt% moisture.

Any amount of said composition is suitable. According to an embodiment, the amount of said composition is at least 100 gram (gr), at least 1 kilogram (Kg), at least 10Kg, at least 100Kg, or at least 1000Kg.

According to an embodiment, said composition, is homogeneous. As used herein, the term homogeneous refers to having a similar content in various samples of the composition. According to an embodiment, a similar content means at least one of a similar size distribution and a similar concentration of at least one major component, at least two, at least three, at least four or at least five major components. As used herein, the term major component refers to a component comprising at least 1wt% of said composition.

According to an embodiment, said major component is a the homogeneous composition, when separated into at least a 10 gram sample, contains a tetrahydrocannabinol concentration in a 1 gram fraction of the at least 10 gram sample, is within 15% of the cannabinoid concentration in a separate 1gram fraction of said 10 gram sample within 13%, within 11%, within 9%, within 7%, within 5%, within 3%, within 2% or within 1%. According to an embodiment said cannabinoid comprises tetrahydrocannabinol (THC). According to another embodiment said cannabinoid comprises cannabidiol (CBD).

According to an embodiment, the size of at least 70wt%, at least 80wt% or at least 90wt% of the comminuted cannabis plant material in a 1 gram fraction of at least 10 gram sample of said homogeneous composition, is within 15% of the size of at least 70wt%, at least 80wt% or at least 90wt% of the comminuted cannabis plant material in a separate 1gram fraction of said 10 gram sample, within 13%, within 11%, within 9%, within 7%, within 5%, within 3%, within 2% or within 1%.

According to an embodiment the homogeneous composition, when separated into at least a 10 gram sample, contains a tetrahydrocannabinol concentration in a 1 gram fraction of the at least 10 gram sample, is within 15% of the tetrahydrocannabinol concentration in a separate 1gram fraction of said 10 gram sample, within 13%, within 11%, within 9%, within 7%, within 5%, within 3%, within 2% or within 1%. According to an embodiment, the cannabidiol concentration in a 1 gram fraction of at least 10 gram sample of said homogeneous composition, is within 15% of the cannabidiol concentration in a separate 1gram fraction of said 10 gram sample, within 13%, within 11%, within 9%, within 7%, within 5%, within 3%, within 2% or within 1%. According to an embodiment, the tetrahydrocannabinol concentration in a 1 gram fraction of at least 10 gram sample of said homogeneous composition, is within 15% of the tetrahydrocannabinol concentration in a separate 1gram fraction of said 10 gram sample, within 13%, within 11%, within 9%, within 7%, within 5%, within 3%, within 2% or within 1% and the cannabidiol concentration in a 1 gram fraction of at least 10 gram sample of said homogeneous composition, is within 15% of the cannabidiol concentration in a separate 1gram fraction of said 10 gram sample, within 13%, within 11%, within 9%, within 7%, within 5%, within 3%, within 2% or within 1%.

According to an embodiment, said homogeneous composition comprises at least 0.5wt% terpene and the terpene concentration in a 1 gram fraction of at least 10 gram sample of said homogeneous composition, is within 15% of the terpene concentration in a separate 1gram fraction of said 10 gram sample within 13%, within 11%, within 9%, within 7%, within 5%, within 3%, within 2% or within 1%.

According to an embodiment, the bulk density of said composition is greater than 0.02 gram/milliliter (gr/ml), greater than 0.04gr/ml, greater than o.o6gr/ml, greater than o.o8gr/ml, greater than 0.1gr/ml or greater than 0.12gr/ml. According to an embodiment, the bulk density of said composition is less than o/6gr/ml, less than 0.5gr/ml, less than 0.4gr/ml, less than 0.3gr/ml, less than 0.25gr/ml or less than 0.2gr/ml.

Further provided is a product containing said homogeneous composition. Further provided is a cannabis cigarette comprising said homogeneous composition. According to an embodiment, said cannabis cigarette burns smoothly during smoking and as a result, according to an embodiment, said cannabis cigarette doesn't require re-lighting during smoking.

According to an embodiment, said cigarette contains at least 0.1gr of said homogeneous composition, at least 0.3gr or at least 0.5gr. According to an embodiment, said cigarette comprises less than 2gr of said homogeneous composition, less than 1.5gr, less than 1.2gr or less than 1gr.

According to an embodiment, said cigarette contains a top fraction and a bottom fraction and the concentration of a major component in a 0.1gram sample from the top fraction of the cigarette is within 15% of the concentration of said major component in a 0.1gram sample from the bottom fraction of the cigarette, within 13%, within 11%, within 9%, within 7%, within 5%, within 3%, within 2% or within 1%.

According to an embodiment, said cigarette contains a top fraction and a bottom fraction and the concentration of tetrahydrocannabinol in a 0.1gram sample from the top fraction of the cigarette is within 15% of tetrahydrocannabinol concentration in a 0.1gram sample from the bottom fraction of the cigarette, within 13%, within 11%, within 9%, within 7%, within 5%, within 3%, within 2% or within 1%. According to an embodiment, said cigarette contains a top fraction and a bottom fraction and the concentration of cannabidiol in a 0.1gram sample from the top fraction of the cigarette is within 15% of cannabidiol concentration in a 0.1gram sample from the bottom fraction of the cigarette, within 13%, within 11%, within 9%, within 7%, within 5%, within 3%, within 2% or within 1%.

According to an embodiment, said cigarette contains a top fraction and a bottom fraction, the concentration of tetrahydrocannabinol in a 0.1gram sample from the top fraction of the cigarette is within 15% of tetrahydrocannabinol concentration in a 0.1gram sample from the bottom fraction of the cigarette, within 13%, within 11%, within 9%, within 7%, within 5%, within 3%, within 2% or within 1% and the concentration of cannabidiol in a 0.1gram sample from the top fraction of the cigarette is within 15% of cannabidiol concentration in a 0.1gram sample from the bottom fraction of the cigarette, within 13%, within 11%, within 9%, within 7%, within 5%, within 3%, within 2% or within 1%.

Further provided is a vaporizer cartridge containing said homogeneous composition.

Further provided is a homogeneous tablet, comprising tetrahydrocannabinol (THC) and cannabidiol (CBD), wherein (i) tetrahydrocannabinol and cannabidiol concentrations are
(a) 0.01-6wt% tetrahydrocannabinol and 0.01-6wt% cannabidiol;
(b) 0.01-6wt% tetrahydrocannabinol and 6-12wt% cannabidiol;
(c) 0.01-6wt% tetrahydrocannabinol and 12-18wt% cannabidiol;
(d) 0.01-6wt% tetrahydrocannabinol and 18-24wt% cannabidiol;
(e) 0.01-6wt% tetrahydrocannabinol and 24-28wt% cannabidiol;
(f) 0.01-6wt% tetrahydrocannabinol and more than 28wt% cannabidiol;
(g) 6-12wt% tetrahydrocannabinol and 0.01-6wt% cannabidiol
(h) 6-12wt% tetrahydrocannabinol and 6-12wt% cannabidiol;
(i) 6-12wt% tetrahydrocannabinol and 12-18wt% cannabidiol;
(j) 6-12wt % tetrahydrocannabinol and 18-24wt% cannabidiol;
(k) 6-12wt % tetrahydrocannabinol and more than 24wt% cannabidiol;
(l) 12-18wt% tetrahydrocannabinol and 0.01-6wt% cannabidiol;
(m) 12-18wt% tetrahydrocannabinol and 6-12wt% cannabidiol;
(n) 12-18wt% tetrahydrocannabinol and 12-18wt% cannabidiol;
(o) 12-18wt% tetrahydrocannabinol and 18-24wt% cannabidiol;
(p) 12-18wt % tetrahydrocannabinol and more than 24wt% cannabidiol;
(q) 18-24wt% tetrahydrocannabinol and 0.01-6wt% cannabidiol;
(r) 18-24wt% tetrahydrocannabinol and 6-12wt% cannabidiol;
(s) 18-24wt% tetrahydrocannabinol and 12-18wt% cannabidiol;
(t) 18-24wt% tetrahydrocannabinol and 18-24wt% cannabidiol;
(u) 18-24wt % tetrahydrocannabinol and more than 24wt% cannabidiol;
(v) More than 24wt% tetrahydrocannabinol and 0.01-6wt%
(w) 24-30wt% tetrahydrocannabinol and 0.01-6wt% cannabidiol;
(x) 24-30wt% tetrahydrocannabinol and 6-12wt% cannabidiol;
(y) 24-30wt% tetrahydrocannabinol and 12-18wt% cannabidiol;
(z) 30-36wt% tetrahydrocannabinol and 0.01-6wt% cannabidiol;
(aa) 30-36wt% tetrahydrocannabinol and 6-12wt% cannabidiol;
(bb) 30-36wt% tetrahydrocannabinol and 12-18wt% cannabidiol;
(cc) More than 36wt% tetrahydrocannabinol and 0.01-3wt% cannabidiol;
(dd) More than 36wt% tetrahydrocannabinol and 3-6wt% cannabidiol; or
(ee) More than 36wt% tetrahydrocannabinol and more than-6wt% cannabidiol;

(ii) said tablet moisture content is greater than 1wt% and less than 20wt% ; (iii) said tablet bulk density is at least 0.1 gram/milliliter and less than 1.2 gram/milliliter;
(iv) the tetrahydrocannabinol concentration in a fraction of said tablet, is within 15% of the tetrahydrocannabinol concentration in a separate fraction of said tablet;
(v) the cannabidiol concentration in a fraction of said tablet, is within 15% of the cannabidiol concentration in a separate fraction of said tablet; and (vi) said tablet comprises at least 70wt% cannabis plant material.

According to an embodiment, said homogeneous tablet comprises 0.01-6wt% tetrahydrocannabinol and 0.01-6wt% cannabidiol. According to an embodiment, said homogeneous tablet comprises at least 0.01wt% tetrahydrocannabinol, at least 2wt% or at least 4wt%. According to an embodiment, said homogeneous composition comprises less than 6wt% tetrahydrocannabinol, less than 4wt% or less than 2wt%. According to an embodiment, said homogeneous composition comprises at least 0.1wt% cannabidiol, at least 2wt% or at least 4wt%. According to an embodiment, said homogeneous composition comprises less than 6wt% cannabidiol, less than 4wt% or less than 2wt%.

According to an embodiment, said homogeneous tablet comprises 0.01-6wt% tetrahydrocannabinol and 6-12wt% cannabidiol. According to an embodiment, said homogeneous tablet comprises at least 0.01wt% tetrahydrocannabinol, at least 2wt% or at least 4wt%. According to an embodiment, said homogeneous tablet comprises less than 6wt% tetrahydrocannabinol, less than 4wt% or less than 2wt%. According to an embodiment, said homogeneous tablet comprises at least 6wt% cannabidiol, at least 8wt% or at least 10wt%. According to an embodiment, said homogeneous tablet comprises less than 12wt% cannabidiol, less than 10wt% or less than 8wt%.

According to an embodiment, said homogeneous tablet comprises 0.01-6wt% tetrahydrocannabinol and 12-18wt% cannabidiol. According to an embodiment, said homogeneous tablet comprises at least 0.1wt% tetrahydrocannabinol, at least 2wt% or at least 4wt%. According to an embodiment, said homogeneous tablet comprises less than 6wt% tetrahydrocannabinol, less than 4wt% or less than 2wt%. According to an embodiment, said homogeneous tablet comprises at least 12wt% cannabidiol, at least 14wt% or at least 16wt%. According to an embodiment, said homogeneous tablet comprises less than 18wt% cannabidiol, less than 16wt% or less than 14wt%.

According to an embodiment, said homogeneous tablet comprises 0.01-6wt% tetrahydrocannabinol and 18-24wt% cannabidiol. According to an embodiment, said homogeneous tablet comprises at least 0.01wt% tetrahydrocannabinol, at least 2wt% or at least 4wt%. According to an embodiment, said homogeneous tablet comprises less than 6wt% tetrahydrocannabinol, less than 4wt% or less than 2wt%. According to an embodiment, said homogeneous tablet comprises at least 18wt% cannabidiol, at least 20wt% or at least 22wt%. According to an embodiment, said homogeneous tablet comprises less than 24wt% cannabidiol, less than 22wt% or less than 20wt%.

According to an embodiment, said homogeneous tablet comprises 0.01-6wt% tetrahydrocannabinol and more than 24wt% cannabidiol.

According to an embodiment, said homogeneous tablet comprises at least 0.01wt% tetrahydrocannabinol, at least 2wt% or at least 4wt%. According to an embodiment, said homogeneous tablet comprises less than 6wt% tetrahydrocannabinol, less than 4wt% or less than 2wt%. According to an embodiment, said homogeneous tablet comprises at least 24wt% cannabidiol, at least 26wt%, at least28wt% or at least 30wt%.

According to an embodiment, said homogeneous tablet comprises 6-12wt% tetrahydrocannabinol and 0.01-6wt% cannabidiol. According to an embodiment, said homogeneous tablet comprises at least 6wt% tetrahydrocannabinol, at least 8wt% or at least 10wt%. According to an embodiment, said homogeneous tablet comprises less than 12wt% tetrahydrocannabinol, less than 10wt% or less than 8wt%. According to an embodiment, said homogeneous tablet comprises at least 0.1wt% cannabidiol, at least 2wt% or at least 4wt%. According to an embodiment, said homogeneous tablet comprises less than 6wt% cannabidiol, less than 4wt% or less than 2wt%.

According to an embodiment, said homogeneous tablet comprises 6-12wt% tetrahydrocannabinol and 6-12wt% cannabidiol. According to an embodiment, said homogeneous tablet comprises at least 6wt% tetrahydrocannabinol, at least 8wt% or at least 10wt%. According to an embodiment, said homogeneous tablet comprises less than 12wt% tetrahydrocannabinol, less than 10wt% or less than 8wt%. According to an embodiment, said homogeneous tablet comprises at least 6wt% cannabidiol, at least 8wt% or at least 10wt%.

According to an embodiment, said homogeneous tablet comprises 6-12wt% tetrahydrocannabinol and 12-18wt% cannabidiol. According to an embodiment, said homogeneous tablet comprises at least 6wt% tetrahydrocannabinol, at least 8wt% or at least 10wt%. According to an embodiment, said homogeneous tablet comprises less than 12wt% tetrahydrocannabinol, less than 10wt% or less than 8wt%. According to an embodiment, said homogeneous tablet comprises at least 12wt% cannabidiol, at least 14wt% or at least 16wt%. According to an embodiment, said homogeneous tablet comprises less than 18wt% cannabidiol, less than 16wt% or less than 14wt%.

According to an embodiment, said homogeneous tablet comprises 6-12wt % tetrahydrocannabinol and 18-24wt% cannabidiol. According to an embodiment, said homogeneous tablet comprises at least 6wt% tetrahydrocannabinol, at least 8wt% or at least 10wt%. According to an embodiment, said homogeneous tablet comprises less than 12wt% tetrahydrocannabinol, less than 10wt% or less than 8wt%. According to an embodiment, said homogeneous tablet comprises at least 18wt% cannabidiol, at least 20wt% or at least 22wt%. According to an embodiment, said homogeneous tablet comprises less than 24wt% cannabidiol, less than 22wt% or less than 20wt%.

According to an embodiment, said homogeneous tablet comprises 6-12wt % tetrahydrocannabinol and more than 24wt% cannabidiol. According to an embodiment, said homogeneous tablet comprises at least 6wt% tetrahydrocannabinol, at least 8wt% or at least 10wt%. According to an embodiment, said homogeneous tablet comprises less than 12wt% tetrahydrocannabinol, less than 10wt% or less than 8wt%. According to an embodiment, said homogeneous tablet comprises at least 24wt% cannabidiol, at least 26wt%, at least28wt% or at least 30wt%.

According to an embodiment, said homogeneous tablet comprises 12-18wt% tetrahydrocannabinol and 0.01-6wt% cannabidiol. According to an embodiment, said homogeneous tablet comprises at least 12wt% tetrahydrocannabinol, at least 14wt% or at least 16wt%. According to an embodiment, said homogeneous tablet comprises less than 18wt% tetrahydrocannabinol, less than 16wt% or less than 14wt%. According to an embodiment, said homogeneous tablet comprises at least 0.1wt% cannabidiol, at least 2wt% or at least 4wt%. According to an embodiment, said homogeneous tablet comprises less than 6wt% cannabidiol, less than 4wt% or less than 2wt%.

According to an embodiment, said homogeneous tablet comprises 12-18wt% tetrahydrocannabinol and 6-12wt% cannabidiol. According to an embodiment, said homogeneous tablet comprises at least 12wt% tetrahydrocannabinol, at least 14wt% or at least 16wt%. According to an embodiment, said homogeneous tablet comprises less than 18wt% tetrahydrocannabinol, less than 16wt% or less than 14wt%. According to an embodiment, said homogeneous tablet comprises at least 6wt% cannabidiol, at least 8wt% or at least 10wt%. According to an embodiment, said homogeneous tablet comprises less than 12wt% cannabidiol, less than 10wt% or less than 8wt%.

According to an embodiment, said homogeneous tablet comprises12-18wt% tetrahydrocannabinol and 12-18wt% cannabidiol. According to an embodiment, said homogeneous tablet comprises at least 12wt% tetrahydrocannabinol, at least 14wt% or at least 16wt%. According to an embodiment, said homogeneous tablet comprises less than 18wt% tetrahydrocannabinol, less than 16wt% or less than 14wt%. According to an embodiment, said homogeneous tablet comprises at least 12wt% cannabidiol, at least 14wt% or at least 16wt%. According to an embodiment, said homogeneous tablet comprises less than 18wt% cannabidiol, less than 16wt% or less than 14wt%.

According to an embodiment, said homogeneous tablet comprises 12-18wt% tetrahydrocannabinol and 18-24wt% cannabidiol. According to an embodiment, said homogeneous tablet comprises at least 12wt% tetrahydrocannabinol, at least 14wt% or at least 16wt%. According to an embodiment, said homogeneous tablet comprises less than 18wt% tetrahydrocannabinol, less than 16wt% or less than 14wt%. According to an embodiment, said homogeneous tablet comprises at least 18wt% cannabidiol, at least 20wt% or at least 22wt%. According to an embodiment, said homogeneous tablet comprises less than 24wt% cannabidiol, less than 22wt% or less than 20wt%.

According to an embodiment, said homogeneous tablet comprises 12-18wt % tetrahydrocannabinol and more than 24wt% cannabidiol. According to an embodiment, said homogeneous tablet comprises at least 12wt% tetrahydrocannabinol, at least 14wt% or at least 16wt%. According to an embodiment, said homogeneous tablet comprises less than 18wt% tetrahydrocannabinol, less than 16wt% or less than 14wt%. According to an embodiment, said homogeneous tablet comprises at least 24wt% cannabidiol, at least 26wt%, at least28wt% or at least 30wt%. According to an embodiment, said homogeneous tablet comprises 18-24wt% tetrahydrocannabinol and 0.1-6wt% cannabidiol. According to an embodiment, said homogeneous tablet comprises at least 18wt% tetrahydrocannabinol, at least 20wt% or at least 22wt%. According to an embodiment, said homogeneous tablet comprises less than 24wt% tetrahydrocannabinol, less than 22wt% or less than 20wt%. According to an embodiment, said homogeneous tablet comprises at least 0.1wt% cannabidiol, at least 2wt% or at least 4wt%. According to an embodiment, said homogeneous tablet comprises less than 6wt% cannabidiol, less than 4wt% or less than 2wt%.

According to an embodiment, said homogeneous tablet comprises 18-24wt% tetrahydrocannabinol and 6-12wt% cannabidiol. According to an embodiment, said homogeneous tablet comprises at least 18wt% tetrahydrocannabinol, at least 20wt% or at least 22wt%. According to an embodiment, said homogeneous tablet comprises less than 24wt% tetrahydrocannabinol, less than 22wt% or less than 20wt%. According to an embodiment, said homogeneous tablet comprises at least 6wt% cannabidiol, at least 8wt% or at least 10wt%. According to an embodiment, said homogeneous tablet comprises less than 12wt% cannabidiol, less than 10wt% or less than 8wt%.

According to an embodiment, said homogeneous tablet comprises 18-24wt% tetrahydrocannabinol and 12-18wt% cannabidiol. According to an embodiment, said homogeneous tablet comprises at least 18wt% tetrahydrocannabinol, at least 20wt% or at least 22wt%. According to an embodiment, said homogeneous tablet comprises less than 24wt% tetrahydrocannabinol, less than 22wt% or less than 20wt%. According to an embodiment, said homogeneous tablet comprises at least 12wt% cannabidiol, at least 14wt% or at least 16wt%. According to an embodiment, said homogeneous tablet comprises less than 18wt% cannabidiol, less than 16wt% or less than 14wt%.

According to an embodiment, said homogeneous tablet comprises 18-24wt% tetrahydrocannabinol and 18-24wt% cannabidiol. According to an embodiment, said homogeneous tablet comprises at least 18wt% tetrahydrocannabinol, at least 20wt% or at least 22wt%. According to an embodiment, said homogeneous tablet comprises less than 24wt% tetrahydrocannabinol, less than 22wt% or less than 20wt%. According to an embodiment, said homogeneous tablet comprises at least 18wt% cannabidiol, at least 20wt% or at least 22wt%. According to an embodiment, said homogeneous tablet comprises less than 24wt% cannabidiol, less than 22wt% or less than 20wt%. According to an embodiment, said homogeneous tablet comprises 18-24wt % tetrahydrocannabinol and more than 24wt% cannabidiol. According to an embodiment, said homogeneous tablet comprises at least 18wt% tetrahydrocannabinol, at least 20wt% or at least 22wt%. According to an embodiment, said homogeneous tablet comprises less than 24wt% tetrahydrocannabinol, less than 22wt% or less than 20wt%. According to an embodiment, said homogeneous tablet comprises at least 24wt% cannabidiol, at least 26wt%, at least28wt% or at least 30wt%.

According to an embodiment, said homogeneous tablet comprises more than 24wt% tetrahydrocannabinol and 0.01-6wt% cannabidiol. According to an embodiment, said homogeneous tablet comprises at least 24wt% tetrahydrocannabinol, at least 26wt%, at least 28wt% or at least 30wt%. According to an embodiment, said homogeneous tablet comprises at least 0.1wt% cannabidiol, at least 2wt% or at least 4wt%. According to an embodiment, said homogeneous tablet comprises less than 6wt% cannabidiol, less than 4wt% or less than 2wt%.

According to an embodiment, said homogeneous tablet comprises more than 24wt% tetrahydrocannabinol and 6-12wt% cannabidiol. According to an embodiment, said homogeneous tablet comprises at least 24wt% tetrahydrocannabinol, at least 26wt%, at least 28wt% or at least 30wt%. According to an embodiment, said homogeneous tablet comprises at least 6wt% cannabidiol, at least 8wt% or at least 10wt%. According to an embodiment, said homogeneous tablet comprises less than 12wt% cannabidiol, less than 10wt% or less than 8wt%.

According to an embodiment, said homogeneous tablet comprises more than 24wt% tetrahydrocannabinol and 12-18wt% cannabidiol. According to an embodiment, said homogeneous tablet comprises at least 24wt% tetrahydrocannabinol, at least 26wt%, at least28wt% or at least 30wt%. According to an embodiment, said homogeneous tablet comprises at least 12 wt% cannabidiol, at least 14wt% or at least 16wt%. According to an embodiment, said homogeneous tablet comprises less than 18wt% cannabidiol, less than 16wt% or less than 14wt%.

According to an embodiment, said homogeneous tablet comprises more than 24wt% tetrahydrocannabinol and 18-24wt% cannabidiol. According to an embodiment, said homogeneous tablet comprises at least 24wt% tetrahydrocannabinol, at least 26wt%, at least28wt% or at least 30wt%. According to an embodiment, said homogeneous tablet comprises at least 18wt% cannabidiol, at least 20wt% or at least 22wt%. According to an embodiment, said homogeneous tablet comprises less than 24wt% cannabidiol, less than 22wt% or less than 20wt%.

According to an embodiment, said homogeneous tablet comprises more than 24wt% tetrahydrocannabinol and more than 24wt% cannabidiol. According to an embodiment, said homogeneous tablet comprises at least 24wt% tetrahydrocannabinol, at least 26wt%, at least28wt% or at least 30wt%. According to an embodiment, said homogeneous tablet comprises at least 24wt% cannabidiol, at least 26wt%, at least28wt% or at least 30wt%.

According to an embodiment, said homogeneous tablet comprises 24-30wt% tetrahydrocannabinol and 0.01-6wt% cannabidiol. According to an embodiment, said homogeneous tablet comprises at least 24wt% tetrahydrocannabinol, at least 26wt%, at least28wt% or at least 30wt%. According to an embodiment, said homogeneous tablet comprises at least 0.01wt% cannabidiol, at least 2wt%, at least4wt% or at least 6wt%.

According to an embodiment, said homogeneous tablet comprises 24-30wt% tetrahydrocannabinol and 6-12wt% cannabidiol. According to an embodiment, said homogeneous tablet comprises at least 24wt% tetrahydrocannabinol, at least 26wt%, at least28wt% or at least 30wt%. According to an embodiment, said homogeneous tablet comprises at least 6wt% cannabidiol, at least 8wt%, at least 10wt% or at least 12wt%.

According to an embodiment, said homogeneous tablet comprises 24-30wt% tetrahydrocannabinol and 12-18wt% cannabidiol. According to an embodiment said homogeneous tablet comprises at least 24wt% tetrahydrocannabinol, at least 26wt%, at least28wt% or at least 30wt%. According to an embodiment, said homogeneous tablet comprises at least 12wt% cannabidiol, at least 14wt%, at least 16wt% or at least 18wt%.

According to an embodiment, said homogeneous tablet comprises 30-36wt% tetrahydrocannabinol and 0.01-6wt% cannabidiol. According to an embodiment, said homogeneous tablet comprises at least 30wt% tetrahydrocannabinol, at least 32wt%, at least 34wt% or at least 36wt%. According to an embodiment, said homogeneous tablet comprises at least 0.01wt% cannabidiol, at least 2wt%, at least4wt% or at least 6wt%.

According to an embodiment, said homogeneous tablet comprises 30-36wt% tetrahydrocannabinol and 6-12wt% cannabidiol. According to an embodiment, said homogeneous tablet comprises at least 30wt% tetrahydrocannabinol, at least 32wt%, at least 34wt% or at least 36wt%. According to an embodiment, said homogeneous tablet comprises at least 6wt% cannabidiol, at least 8wt%, at least 10wt% or at least 12wt%.

According to an embodiment, said homogeneous tablet comprises 30-36wt% tetrahydrocannabinol and 12-18wt% cannabidiol. According to an embodiment, said homogeneous tablet comprises at least 30wt% tetrahydrocannabinol, at least 32wt%, at least 34wt% or at least 36wt%. According to an embodiment, said homogeneous tablet comprises at least 12wt% cannabidiol, at least 14wt%, at least 16wt% or at least 18wt%.

According to an embodiment, said homogeneous tablet comprises more than 36wt% tetrahydrocannabinol and 0.01-6wt% cannabidiol. According to an embodiment, said homogeneous tablet comprises at least 0.01wt% cannabidiol, at least 2wt%, at least4wt% or at least 6wt%.

According to an embodiment, said homogeneous tablet comprises more than 36wt% tetrahydrocannabinol and 6-12wt% cannabidiol. According to an embodiment, said homogeneous tablet comprises at least 6wt% cannabidiol, at least 8wt%, at least 10wt% or at least 12wt%.

According to an embodiment said homogeneous tablet comprises more than 36wt% tetrahydrocannabinol and 12-18wt% cannabidiol. According to an embodiment, said homogeneous tablet comprises at least 12wt% cannabidiol, at least 14wt%, at least 16wt% or at least 18wt%.

According to an embodiment, said homogeneous tablet comprises more than 36wt% tetrahydrocannabinol and more then 6wt% cannabidiol.

According to an embodiment, said homogeneous tablet comprises more than 36 wt% tetrahydrocannabinol and 0.01-9wt% cannabidiol. According to an embodiment, said homogeneous tablet comprises at least 36wt% tetrahydrocannabinol. According to an embodiment, said homogeneous tablet comprises at least 0.01wt% cannabidiol, at least 3wt%, at least 5% or at least 9wt%. According to an embodiment, said homogeneous tablet comprises less than 9wt% cannabidiol, less than 5wt% or less than 3wt%.

According to an embodiment, said tablet composition comprises 30-36wt% tetrahydrocannabinol and 0.01-15wt% cannabidiol. According to an embodiment, said homogeneous tablet comprises at least 32wt% tetrahydrocannabinol, at least 34wt% or at least 36wt%. According to an embodiment, said homogeneous tablet comprises less than 36wt% tetrahydrocannabinol, less than 34wt% or less than 32wt%. According to an embodiment, said homogeneous tablet comprises at least 0.01wt% cannabidiol, at least 7wt% or at least 15wt%. According to an embodiment, said homogeneous tablet comprises less than 15wt% cannabidiol, less than 10wt% or less than 3wt%.

According to an embodiment, said homogeneous tablet comprises 24-30wt% tetrahydrocannabinol and 0.01-18wt% cannabidiol. According to an embodiment, said homogeneous tablet comprises at least 26wt% tetrahydrocannabinol, at least 28wt% or at least 30wt%. According to an embodiment, said homogeneous tablet comprises less than 30wt% tetrahydrocannabinol, less than 28wt% or less than 26wt%. According to an embodiment, said homogeneous tablet comprises at least 0.01wt% cannabidiol, at least 10wt% or at least 16wt%. According to an embodiment, said homogeneous tablet comprises less than 18wt% cannabidiol, less than 10wt% or less than 3wt%.

According to an embodiment, said homogeneous tablet comprises 18-24wt% tetrahydrocannabinol and 0.01-18wt% cannabidiol. According to an embodiment, said homogeneous tablet comprises at least 20wt% tetrahydrocannabinol, at least 22wt% or at least 24wt%. According to an embodiment, said homogeneous tablet comprises less than 26wt% tetrahydrocannabinol, less than 24wt% or less than 22wt%. According to an embodiment, said tablet composition comprises at least o.oiwt% cannabidiol, at least 10wt% or at least 16wt%. According to an embodiment, said homogeneous tablet comprises less than 18wt% cannabidiol, less than 10wt% or less than 3wt%.

According to an embodiment, said homogeneous tablet comprises 12-18wt% tetrahydrocannabinol and 0.01-21wt% cannabidiol. According to an embodiment, said homogeneous tablet comprises at least 12wt% tetrahydrocannabinol, at least 14wt% or at least 16wt%. According to an embodiment, said homogeneous tablet comprises less than 18wt% tetrahydrocannabinol, less than 16wt% or less than 14wt%. According to an embodiment, said homogeneous tablet comprises at least 0.01wt% cannabidiol, at least 10wt% or at least 20wt%. According to an embodiment, said homogeneous tablet comprises less than 21wt% cannabidiol, less than 10wt% or less than 3wt%.

According to an embodiment, said homogeneous tablet comprises 6-12wt% tetrahydrocannabinol and 0.01-24wt% cannabidiol. According to an embodiment, said homogeneous tablet comprises at least 6wt% tetrahydrocannabinol, at least 8wt% or at least 10wt%. According to an embodiment, said homogeneous tablet comprises less than 12wt% tetrahydrocannabinol, less than 10wt% or less than 8wt%. According to an embodiment, said homogeneous tablet comprises at least 0.01wt% cannabidiol, at least 10wt% or at least 22wt%. According to an embodiment, said homogeneous tablet comprises less than 24wt% cannabidiol, less than 10wt% or less than 3wt%.

According to an embodiment, said homogeneous tablet comprises 0.01-6wt% tetrahydrocannabinol and 0.01-34wt% cannabidiol. According to an embodiment, said homogeneous tablet comprises at least 0.01wt% tetrahydrocannabinol, at least 2wt% or at least 4wt%. According to an embodiment, said homogeneous tablet comprises less than 6wt% tetrahydrocannabinol, less than 4wt% or less than 2wt%. According to an embodiment, said homogeneous tablet comprises at least 0.01wt% cannabidiol, at least 15wt% or at least 30wt%. According to an embodiment, said homogeneous tablet comprises less than 34wt% cannabidiol, less than 20wt% or less than 10wt%.

According to an embodiment, said homogeneous tablet comprises 16-24wt% tetrahydrocannabinol and 1-7wt% cannabidiol. According to an embodiment, said homogeneous tablet comprises at least 16wt% tetrahydrocannabinol, at least 18wt% or at least 20wt%. According to an embodiment, said homogeneous tablet comprises less than 24wt% tetrahydrocannabinol, less than 22wt% or less than 20wt%. According to an embodiment, said homogeneous tablet comprises at least 1wt% cannabidiol, at least 3wt% or at least 5wt%. According to an embodiment, said homogeneous tablet comprises less than 7wt% cannabidiol, less than 5wt% or less than 3wt%.

According to an embodiment, said homogeneous tablet comprises 11-19wt% tetrahydrocannabinol and 0.05-5.5wt% cannabidiol. According to an embodiment, said homogeneous tablet comprises at least 11wt% tetrahydrocannabinol, at least 13wt% or at least 15wt%. According to an embodiment, said homogeneous tablet comprises less than 19wt% tetrahydrocannabinol, less than 17wt% or less than 15wt%. According to an embodiment, said homogeneous tablet comprises at least 0.5wt% cannabidiol, at least 1.5wt% or at least 2.5wt%. According to an embodiment, said homogeneous tablet comprises less than 5.5wt% cannabidiol, less than 4.5wt% or less than 3.5wt%.

According to an embodiment, said homogeneous tablet comprises 6-14wt% tetrahydrocannabinol and 0.02-3.8wt% cannabidiol. According to an embodiment, said homogeneous tablet comprises at least 6wt% tetrahydrocannabinol, at least 8wt% or at least 10wt%. According to an embodiment, said homogeneous tablet comprises less than 14wt% tetrahydrocannabinol, less than 12wt% or less than 10wt%. According to an embodiment, said homogeneous tablet comprises at least 0.5wt% cannabidiol, at least 1wt% or at least 1.5wt%. According to an embodiment, said homogeneous tablet comprises less than 3.5wt% cannabidiol, less than 2.5wt% or less than 1.5wt%.

According to an embodiment, said homogeneous tablet comprises 6-14wt% tetrahydrocannabinol and 6-14wt% cannabidiol. According to an embodiment, said homogeneous tablet comprises at least 6wt% tetrahydrocannabinol, at least 8wt% or at least 10wt%. According to an embodiment, said homogeneous tablet comprises less than 14wt% tetrahydrocannabinol, less than 12wt% or less than 10wt%. According to an embodiment, said homogeneous tablet comprises at least 6wt% cannabidiol, at least 8wt% or at least 10wt%. According to an embodiment, said homogeneous tablet comprises less than 14wt% cannabidiol, less than 12wt% or less than 10wt%.

According to an embodiment, said homogeneous tablet comprises 2.5-7.5wt% tetrahydrocannabinol and 6-14wt% cannabidiol. According to an embodiment, said homogeneous tablet comprises at least 2.5wt% tetrahydrocannabinol, at least 3.5wt% or at least 4.5wt%. According to an embodiment, said homogeneous tablet comprises less than 7.5wt% tetrahydrocannabinol, less than 6.5wt% or less than 5.5wt%. According to an embodiment, said homogeneous tablet comprises at least 6wt% cannabidiol, at least 8wt% or at least 10wt%. According to an embodiment, said homogeneous tablet comprises less than 14wt% cannabidiol, less than 12wt% or less than 10wt%.

According to an embodiment, said homogeneous tablet comprises 0.05-5.5wt% tetrahydrocannabinol and 11-19wt% cannabidiol. According to an embodiment, said homogeneous tablet comprises at least 0.05wt% tetrahydrocannabinol, at least 1.5wt% or at least 2.5wt%. According to an embodiment, said homogeneous tablet comprises less than 5.5wt% tetrahydrocannabinol, less than 4.5wt% or less than 3.5wt%. According to an embodiment, said homogeneous tablet comprises at least 11wt% cannabidiol, at least 13wt% or at least 15wt%. According to an embodiment, said homogeneous tablet comprises less than 19wt% cannabidiol, less than 17wt% or less than 15wt%.

According to an embodiment, said homogeneous tablet comprises 0.01-2.5wt% tetrahydrocannabinol and 16-24wt% cannabidiol. According to an embodiment, said homogeneous tablet comprises at least 0.05wt% tetrahydrocannabinol, at least 1wt% or at least 1.5wt%. According to an embodiment, said homogeneous tablet comprises less than 2.5wt% tetrahydrocannabinol, less than 2wt% or less than 1.5wt%. According to an embodiment, said homogeneous tablet comprises at least 16wt% cannabidiol, at least 18wt% or at least 20wt%. According to an embodiment, said homogeneous tablet comprises less than 24wt% cannabidiol, less than 22wt% or less than 20wt%.

According to an embodiment, said homogeneous tablet comprises 0.01-0.05wt% tetrahydrocannabinol and 20-28wt% cannabidiol. According to an embodiment, said homogeneous tablet comprises at least 20wt% cannabidiol, at least 22wt% or at least 24wt%. According to an embodiment, said homogeneous composition comprises less than 28wt% cannabidiol, less than 26wt% or less than 24wt%.

According to an embodiment, said homogeneous tablet, comprises less than 1wt% cannabinol, less than 0.8wt%, less than 0.7wt%, less than 0.6wt% or less than 0.5wt%.

According to an embodiment, the tetrahydrocannabinol comprises tetrahydrocannabinol and tetrahydrocannabinolic acid and the weight per weight ratio between tetrahydrocannabinol and tetrahydrocannabinolic acid in said tablet is in the range between 20 to 1 and 1 to 20. According to an embodiment, the weight per weight ratio between tetrahydrocannabinol and tetrahydrocannabinolic acid in said tablet is less than 18:1, less than 16:1, less than 14:1, less than 12:1, less than 10:1, less than 8:1, less than 6:1, less than 4:1, less than 2:1 or less than 1:1. According to an embodiment, the weight per weight ratio between tetrahydrocannabinol and tetrahydrocannabinolic acid in said tablet is greater than 1:20, greater than 1:18, greater than 1:16, greater than 1:14, greater than 1:12, greater than 1:10, greater than 1:8, greater than 1:6, greater than 1:4, greater than 1:2, or greater than 1:1.

According to an embodiment, the cannabinol comprises cannabinol and cannabinolic acid and the weight per weight ratio between cannabidiol and cannabidiolic acid in said tablet is in the range between 20 to 1 and 1 to 20. According to an embodiment, the weight per weight ratio between cannabidiol and cannabidiolic acid in said tablet is less than 18:1, less than 16:1, less than 14:1, less than 12:1, less than 10:1, less than 8:1, less than 6:1, less than 4:1, less than 2:1 or less than 1:1. According to an embodiment, the weight per weight ratio between cannabidiol and cannabidiolic acid in said tablet is greater than 1:20, greater than 1:18, greater than 1:16, greater than 1:14, greater than 1:12, greater than 1:10, greater than 1:8, greater than 1:6, greater than 1:4, greater than 1:2, or greater than 1:1.

According to an embodiment at least 70wt% of the tablet comprises cannabis plant material, at least 75wt%, least 80wt%, least 85wt%, least 90wt% or at least 95wt%. According to an embodiment at least 70wt% of the cannabis plant material in said tablet is derived from cannabis flower (also referred to as cannabis bud), at least 75wt%, least 80wt%, least 85wt%, least 90wt% or at least 95wt%. As used herein, derived from cannabis flower refers to being cannabis flower or being a product or processing cannabis flower, e.g. drying and/or comminuting a cannabis flower. According to an embodiment, at least a fraction of said cannabis plant material in said composition comprises dried plant material.

According to an embodiment said tablet comprises moisture. According to an embodiment, moisture content can be determined loss on drying. According to an embodiment said composition comprises at least 1wt% moisture; at least 3wt%, at least 5wt%, at least 7wt%, at least 9wt%, or at least 11wt%. According to an embodiment said composition comprises less than 20wt% moisture, less than 18wt%, less than 16wt%, or less than 14wt%. According to an embodiment said composition comprises between 10wt% and 14wt% moisture or between 11wt% and 13wt% moisture.

According to an embodiment, the bulk density of said cannabis tablet is greater than 0.1gr/ml, greater than 0.3gr/ml, greater than 0.5gr/ml, greater than o.6gr/ml, greater than 0.7gr/ml, greater than o.8gr/ml or greater than 0.85gr/ml. According to an embodiment, the bulk density of said cannabis tablet is less than 1.2gr/ml, less than 1.1gr/ml, less than 1.05gr/ml, less than 1.0gr/ml, less than 0.95gr/ml or less than 0.90gr/ml.

According to an embodiment, said cannabis tablet, is homogeneous. As used herein, the term homogeneous refers to having similar content in various fractions of the tablet. According to an embodiment, similar content is determined by chemical analysis of a major component. According to an embodiment, the tetrahydrocannabinol concentration in a fraction of said tablet, is within 15% of the tetrahydrocannabinol concentration in a separate fraction of said tablet within 13%, within 11%, within 9%, within 7%, within 5%, within 3%, within 2% or within 1%. According to an embodiment, the cannabidiol concentration in a fraction of said tablet, is within 15% of the cannabidiol concentration in a separate fraction of said tablet, within 13%, within 11%, within 9%, within 7%, within 5%, within 3%, within 2% or within 1%.

According to an embodiment, the weight of said tablet is between 0.1gr and 2gr, between 0.2gr and 1.5gr or between 0.25gr and 1gr.

Further provided is a method for producing a homogeneous comminuted cannabis plant material composition, comprising
(i)providing a tetrahydrocannabinol-comprising cannabis plant material;
(ii) providing a cannabidiol-comprising cannabis plant material;
(iii) (a) comminuting each one of said tetrahydrocannabinol-comprising cannabis plant materials, and cannabidiol-comprising cannabis plant material whereby two separate comminuted homogeneous cannabis plant materials are formed, and/or (b) comminuting one of said tetrahydrocannabinol-comprising cannabis plant materials, and cannabidiol-comprising cannabis plant material, whereby a comminuted homogeneous cannabis plant materials is formed and extracting the non-comminuted cannabis plant material to form a cannabinoid-comprising extract; and
(iv) (a) blending said two separate comminuted homogeneous cannabis plant materials of step (iii)(a) in a proportion calculated to form the said composition according to Claim 1, and/or (b) blending said comminuted homogeneous cannabis plant material and said cannabinoid-comprising extract of step (iii)(b) in a proportion to form the homogeneous comminuted cannabis plant material composition described above

According to an embodiment said provided cannabis plant material comprises less than 30wt% moisture, less than 28wt%, less than 26wt%, less than 24wt%, less than 22wt%, less than 20wt%, less than 18wt%, less than 16wt%, or less than 14wt%. According to an embodiment said providing cannabis plant material comprises at least 1wt% moisture; at least 3wt%, at least 5wt%, at least 7wt%, at least 9wt%, or at least 11wt%.

According to an embodiment, said comminuting comprises maintaining said provided cannabis plant material at a temperature of less than 0°C for at least 1 hour to form a frozen cannabis plant material. According to an embodiment said provided cannabis plant material in kept at a temperature of less than 0°C, less than minus 5°C, less than minus 10°C, less than minus 15°C or less than minus 20°C. According to an embodiment said provided cannabis plant material in kept at a that temperature for at least 1hour (hr) to form frozen plant material, for at least 2hr, at least 4hr, at least 6hr, at least 8hr, at least 10hr, at least 12hr, at least 14hr or at least 16hr.

According to an embodiment, said comminuting comprises comminuting said frozen cannabis plant material by pressing against a mesh screen with a sieve size smaller than 4.2 millimeters to form expelled homogeneous composition comprising comminuted cannabis plant material. According to an embodiment, said screen is shaped as a drum or a fraction of the drum. According to an embodiment, pressing is done by an oscillating unit within said drum. According to an embodiment, oscillation is conducted at a rate between 30 and 500 rounds per minute (RPM), between 50 and 450RPM or between 100 and 400RPM.

According to an embodiment, screen pore size is less than 4.0mm, less than 3.8mm, less than 3.6mm, less than 3.4mm, less than 3.2mm or less than 3.0mm. According to an embodiment, screen pore size is at least 0.3mm, at least 0.6mm, at least 0.8mm, at least 1.0mm, at least 1.2mm, at least 1.4mm, at least 1.6mm, at least 1.8mm or at least 2.0mm.

According to an embodiment, said comminuting comprises collecting said expelled, homogeneous composition. Any form of collecting is suitable.

According to an embodiment said method comprises analyzing a sample of said collected expelled homogeneous composition. According to an embodiment said method comprises analyzing said comminuted homogeneous cannabis plant material and said cannabinoid-comprising extract and blending them in a proportion calculated to form the comminuted cannabis plant material composition.

According to an embodiment, said extracting comprises (i) optionally comminuting the non-comminuted cannabis plant material of step (iii)(b), whereby comminuted cannabis plant material is formed; (ii) contacting said non-comminuted cannabis plant material or said comminuted cannabis plant material with a liquid extractant for at least 1 minute to form cannabinoid-depleted plant material and a cannabinoid-comprising extractant; (iii) separating said cannabinoid-depleted plant material from said cannabinoid-comprising extractant to form separated cannabinoid-comprising extractant.

According to an embodiment, contacting said provided cannabis plant material or said comminuted cannabis plant material with a liquid extractant for at least 1 minute, at least 3 minutes, at least 5 minutes to form cannabinoid-depleted plant material and a cannabinoid-comprising extractant.

According to an embodiment said method comprises analyzing a sample of said cannabinoid-comprising extractant. According to an embodiment said method comprises analyzing said two separate comminuted homogeneous cannabis plant materials and blending them in a proportion calculated to form the comminuted cannabis plant material composition.

According to an embodiment, said method comprises comminuting both said cannabis plant materials and_blending said two comminuted cannabis plant materials with said cannabinoid-depleted plant material in a proportion calculated to form the said composition.

According to an embodiment, said method comprises blending said comminuted cannabis plant material and said cannabinoid-comprising extract with said cannabinoid-depleted plant material in a proportion calculated to form the said composition.

According to an embodiment at least 70wt% of said provided a tetrahydrocannabinol-comprises cannabis plant material is derived from cannabis plant flower, at least 75wt%, least 80wt%, least 85wt%, least 90wt% or at least 95wt%. According to an embodiment at least 70wt% of said provided a cannabidiol-comprising cannabis plant material is derived from cannabis plant flower, at least 75wt%, least 80wt%, least 85wt%, least 90wt% or at least 95wt%.

According to an embodiment said provided tetrahydrocannabinol-comprises cannabis plant material and/or said provided cannabidiol-comprising cannabis plant material comprise moisture at more than 1wt%.

Any rate of production is applicable. According to an embodiment, said homogeneous composition is produced at a rate of at least 1Kg/hr, at least 10Kg/hr, at least 100Kg/hr or at least 1000Kg/hr.

According to an embodiment, said method further comprises adding said homogeneous composition to a cigarettes tube or a cigarette paper, whereby a cigarette is formed. According to an embodiment, said method comprises producing at least 10 cigarettes per hour, at least 100, at least 1000 or at least 10,000.

According to an embodiment, further provided is a cannabis cigarette produced according to the method of the present invention. According to an embodiment, said cigarette contains a top fraction and a bottom fraction and the concentration of a major component in a 0.1gram sample from the top fraction of the cigarette is within 15% of the concentration of said major component in a 0.1gram sample from the bottom fraction of the cigarette, within 13%, within 11%, within 9%, within 7%, within 5%, within 3%, within 2% or within 1%. According to an embodiment, said cigarette contains a top fraction and a bottom fraction and the concentration of tetrahydrocannabinol in a 0.1gram sample from the top fraction of the cigarette is within 15% of tetrahydrocannabinol concentration in a 0.1gram sample from the bottom fraction of the cigarette, within 13%, within 11%, within 9%, within 7%, within 5%, within 3%, within 2% or within 1%. According to an embodiment, said cigarette contains a top fraction and a bottom fraction and the concentration of cannabidiol in a 0.1gram sample from the top fraction of the cigarette is within 15% of cannabidiol concentration in a 0.1gram sample from the bottom fraction of the cigarette, within 13%, within 11%, within 9%, within 7%, within 5%, within 3%, within 2% or within 1%.

According to an embodiment, said cigarette contains a top fraction and a bottom fraction, the concentration of tetrahydrocannabinol in a 0.1gram sample from the top fraction of the cigarette is within 15% of tetrahydrocannabinol concentration in a 0.1gram sample from the bottom fraction of the cigarette, within 13%, within 11%, within 9%, within 7%, within 5%, within 3%, within 2% or within 1% and the concentration of cannabidiol in a 0.1gram sample from the top fraction of the cigarette is within 15% of cannabidiol concentration in a 0.1gram sample from the bottom fraction of the cigarette, within 13%, within 11%, within 9%, within 7%, within 5%, within 3%, within 2% or within 1%.

According to an embodiment, further provided is a batch of cannabis cigarettes produced according to said method, e.g. 100 cigarettes, 500 cigarettes, 1000 cigarettes, 5000 cigarettes, or 10000 cigarettes, and the content of tetrahydrocannabinol in one of said cigarettes is within 15% of the tetrahydrocannabinol content in a separate cigarette, within13%, within 11%, within 9%, within 7%, within 5%, within 3%, within 2% or within 1%. According to an embodiment, the content of cannabidiol in one of said cigarettes is within 15% of the cannabinoid content in a separate cigarette, within13%, within 11%, within 9%, within 7%, within 5%, within 3%, within 2% or within 1%.

According to an embodiment, further provided is a batch of cannabis cigarettes produced according to said method, e.g. 100 cigarettes, 500 cigarettes, 1000 cigarettes, 5000 cigarettes, or 10000 cigarettes, and the content of tetrahydrocannabinol in one third of the batch of the cigarettes is within 15% of the tetrahydrocannabinol content in a separate third of the batch of the cigarettes, within13%, within 11%, within 9%, within 7%, within 5%, within 3%, within 2% or within 1%. According to an embodiment, further provided is a batch of cannabis cigarettes produced according to said method, e.g. 100 cigarettes, 500 cigarettes, 1000 cigarettes, 5000 cigarettes, or 10000 cigarettes, and the content of cannabidiol in one third of the batch of the cigarettes is within 15% of the cannabidiol content in a separate third of the batch of the cigarettes, within13%, within 11%, within 9%, within 7%, within 5%, within 3%, within 2% or within 1%.

According to an embodiment, said method further comprises pressing said collected homogeneous cannabis plant material to form a homogeneous cannabis tablet. According to an embodiment, said pressing is conducted in a tablet-making machine. According to an embodiment, said method comprises producing at least 10 tablets per minute, at least 100, at least 1000 or at least 10,000.

According to an embodiment, the bulk density of said formed cannabis tablet is greater than 0.1gr/ml, greater than 0.3gr/ml, greater than 0.5gr/ml, greater than o.6gr/ml, greater than 0.7gr/ml, greater than o.8gr/ml or greater than 0.85gr/ml. According to an embodiment, the bulk density of said formed cannabis tablet is less than 1.2gr/ml, less than 1.1gr/ml, less than 1.05gr/ml, less than 1.0gr/ml, less than 0.95gr/ml or less than 0.90gr/ml.

According to an embodiment, tetrahydrocannabinol concentration in a fraction of said tablet, is within 15% of the tetrahydrocannabinol concentration in a separate fraction of said tablet; within 13%, within 11%, within 9%, within 7%, within 5%, within 3%, within 2% or within 1%. According to an embodiment, the cannabidiol concentration in a fraction of said tablet, is within 15% of the cannabidiol concentration in a separate fraction of said tablet, within 13%, within 11%, within 9%, within 7%, within 5%, within 3%, within 2% or within 1%.

According to an embodiment, further provided is a batch of at least 10 homogeneous cannabis tablets produced according to said method, at least 100 or at least 1000, wherein tetrahydrocannabinol content in one tablet is within 15% of the tetrahydrocannabinol content in a separate tablet, within 13%, within 11%, within 9%, within 7%, within 5%, within 3%, within 2% or within 1%.

According to an embodiment, further provided is a batch of at least 10 homogeneous cannabis tablets produced according to said method, at least 100 or at least 1000, wherein cannabidiol content in one tablet is within 15% of the cannabidiol content in a separate tablet, within 13%, within 11%, within 9%, within 7%, within 5%, within 3%, within 2% or within 1%. According to an embodiment, further provided is a batch of homogeneous cannabis tablets produced according to said method, wherein cannabinoid content in one third of the tablets is within 15% of the THC content in a separate third of the tablets, within 13%, within 11%, within 9%, within 7%, within 5%, within 3%, within 2% or within 1%.

According to an embodiment, the weight of said tablet is at least 0.05gr, at least 0.1gr or at least0.15gr. According to an embodiment, the weight of said tablet is less than 1gr, less than 0.8gr, less than 0.6gr or less than 0.4gr. According to an embodiment, the cannabinoid content in said tablet is at least 2 milligrams (mg), at least 5mg, at least 10mg or at least 15mg. According to an embodiment, the cannabinoid content in said tablet is less than 50mg, less than 40mg, less than 30mg, or less than 20mg.

According to an embodiment, the method further comprises heating said cannabis tablet to a temperature greater than 100°C, whereby said cannabinoid decarboxlates and/or evaporate. According to an embodiment, said heating decarboxylation and evaporation are conducted in a cannabis vaporizer.

### Examples

Examples 1: Production of homogeneous comminuted cannabis composition via blending two separate homogeneous comminuted cannabis plant materials.

Tetrahydrocannabinol-comprising cannabis flowers were dried to moisture content of 14wt%. Separately, cannabidiol-comprising cannabis flowers were dried to moisture content of 14wt%. The dried flowers from both plants were kept in a freezer at a temperature of minus 10°C for 24 hours. The frozen flowers of each plant were comminuted, as such, without warming, by pressing against a screen with a pore size of 2mm. The expelled comminuted material was collected and allowed to warm to (plus) 20°C. Then a sample of the expelled material was taken and its bulk density was determined. Bulk densities of 0.138 and 0.143 (gr/ ml) were measured for the tetrahydrocannabinol-comprising cannabis and cannabidiol-comprising cannabis comminuted plant materials respectively.

The two comminuted homogeneous cannabis plant materials were blended at a proportion calculated to form 18% THC 6% CBD homogeneous comminuted cannabis plant material composition.

Example 2: Flowers of tetrahydrocannabinol-comprising cannabis plant A were dried to a moisture content of 12wt%. Separately Flowers of cannabidiol-comprising cannabis plant B were dried to a moisture content of 12wt%. The dried flowers from each plant were kept in a freezer at a temperature of minus 10°C for 24 hours. The frozen flowers of plant A were comminuted, as such, without warming, by pressing against a screen with a pore size of 2mm. The expelled comminuted material was collected, allowed to warm up to (plus) 20°C and homogenized to form comminuted homogeneous flower material A. The frozen flowers of plant B were treated similarly to form comminuted homogeneous flower material B. Each flower material was analyzed for their cannabinoids and terpenes content.

Examples 3-10: Flowers of tetrahydrocannabinol-comprising cannabis plant A were dried to a moisture content of 12wt%. Separately, flowers of cannabidiol-comprising cannabis plant B were dried to a moisture content of 12wt%. The dried flowers from each plant were kept in a freezer at a temperature of minus 10°C for 24 hours. The frozen flowers of plant A were comminuted, as such, without warming, by pressing against a screen with a pore size of 2mm. The expelled comminuted material was collected, allowed to warm up to (plus) 20°C and homogenized to form comminuted homogeneous flower material A. The frozen flowers of plant B were treated similarly to form comminuted homogeneous flower material B. Each flower material was analyzed for its cannabinoids and terpenes content. Selected amounts of flower material A were blended with selected amounts of flower material B to form homogeneous blends. The blend was analyzed for its cannabinoids and terpenes content. Results are summarized in the following Table 1. A large variety of compositions was formed through that blending.

**Table 1**

| Example | Flower material A cannabinoids content (%) | | Flower material B cannabinoids content (%) | | Selected amounts (Kg) | | Blend cannabinoids content (%) | |
|---|---|---|---|---|---|---|---|---|
| | THCa | CBDa | THCa | CBDa | A | B | THCa | CBDa |
| 3 | 24.2 | 0.1 | 11.8 | 9.6 | 0.5 | 1.5 | 15 | 7.1 |
| 4 | 24.2 | 0.1 | 11.8 | 9.6 | 1.5 | 0.5 | 21 | 4.9 |
| 5 | 15.3 | 0.1 | 7.8 | 13.3 | 0.5 | 1.5 | 9.2 | 10 |
| 6 | 15.3 | 0.1 | 7.8 | 13.3 | 1.5 | 0.5 | 13.3 | 3.3 |
| 7 | 18.4 | 0.1 | 3.2 | 15.5 | 0.5 | 1.5 | 7 | 11.6 |
| 8 | 18.4 | 0.1 | 3.2 | 15.5 | 1.5 | 0.5 | 14.4 | 3.9 |
| 9 | 8.8 | 12.2 | 14.8 | 10.2 | 0.5 | 1.5 | 13.3 | 10.6 |
| 10 | 8.8 | 12.2 | 14.8 | 10.2 | 1.5 | 0.5 | 10.2 | 12 |

Examples 11 - 17: Flowers of cannabis plant A were dried to a moisture content of 12wt%. Separately, flowers of cannabis plant B were dried to a moisture content of 12wt%. The dried flowers from each plant were kept in a freezer at a temperature of minus 10°C for 24 hours. The frozen flowers of plant A were comminuted, as such, without warming, by pressing against a screen with a pore size of 2mm. The expelled comminuted material was collected, allowed to warm up to (plus) 20°C and homogenized to form comminuted homogeneous flower material A. This flower material A was analyzed for its cannabinoids and terpenes content. The frozen flowers of plant B were treated similarly to form comminuted homogeneous flower material B. A selected weight of flower material B was mixed at ambient temperature in an extraction vessel with 10 weights of 99% ethanol for 30 minutes. Then the residual flower material was filtered out. The remaining, B extract-containing ethanol was evaporated to remove 80% of its volume, forming concentrated B extract. A sample of that concentrated B extract was subjected to further evaporation to form desolventized B extract. That desolventized B extract was analyzed for its cannabinoids content. A selected amount of flower material A was thoroughly mixed with a selected amount of concentrated B extract to form a wet A+B blend. That wet blend was dried in an oven at 40°C to form a dry A+B blend. That dry blend was analyzed for its cannabinoids content. Results are summarized in the following Table 2. Large variety of dry blends was formed, including dry blends with cannabinoids concentrations far beyond those found in cannabis flowers.

**Table 2**

| Example | Flower material A cannabinoids content (%) | | Desolventized B extract cannabinoids content (%) | | Selected amounts (Kg) | | Dry A+B blend cannabinoids content (%) | |
|---|---|---|---|---|---|---|---|---|
| | THCa | CBDa | THCa | CBDa | A | B | THCa | CBDa |
| 11 | 24.2 | 0.1 | 67 | 1 | 1 | 0.35 | 35 | 0.4 |
| 12 | 1 | 16 | 4 | 64 | 1 | 0.35 | 1.5 | 28 |
| 13 | 24.2 | 0.1 | 30 | 34 | 1 | 0.25 | 25 | 7 |
| 14 | 7.8 | 13.3 | 4 | 64 | 1 | 0.3 | 7 | 25 |
| 15 | 7.8 | 13.3 | 30 | 34 | 1 | 0.3 | 13 | 18 |
| 16 | 7.8 | 13.3 | 67 | 1 | 1 | 0.3 | 21 | 10.5 |
| 17 | 14.8 | 10.2 | 30 | 34 | 1 | 0.3 | 18 | 16 |

Examples 18 - 24: Flowers of cannabis plant A were dried to a moisture content of 12wt%. Separately, flowers of cannabis plant B were dried to a moisture content of 12wt%. The dried flowers from each plant were kept in a freezer at a temperature of minus 10°C for 24 hours. The frozen flowers of plant A were comminuted, as such, without warming, by pressing against a screen with a pore size of 2mm. The expelled comminuted material was collected, allowed to warm up to (plus) 20°C and homogenized to form comminuted homogeneous flower material A. This flower material A was analyzed for its cannabinoids and terpenes content. The frozen flowers of plant B were treated similarly to form comminuted homogeneous flower material B. A selected weight of flower material B was mixed at ambient temperature in an extraction vessel with 10 weights of 99% ethanol for 30 minutes. Then, the residual flower material was filtered out. The remaining, B extract-containing ethanol was evaporated to remove all ethanol content and then kept at 120 °C to complete decarboxylation, forming decarboxylated B extract. A sample of that decarboxylated B extract was analyzed for its cannabinoids content. The decarboxylated B extract was diluted by mixing each weight of it with two weights of ethanol 99% to form diluted decarboxylated B extract. A selected amount of flower material A was thoroughly mixed with a selected amount of diluted decarboxylated B extract to form a wet A+B(d) blend. That wet blend was dried in an oven at 40°C to form a dry A+B(d) blend. That dry blend was analyzed for its cannabinoids content. Results are summarized in the following Table 3. Large variety of dry blends was formed, including dry blends with cannabinoids concentrations far beyond those found in cannabis flowers and various ratios between decarboxylated and non-decarboxylated cannabinoids.

**Table 3**

| Ex. | Flower material A cannabinoids content (%) | | Decarboxylated B extract cannabinoids content (%) | | Selected amounts (Kg) | | Dry A+B(d) blend cannabinoids content (%) | | | |
|---|---|---|---|---|---|---|---|---|---|---|
| | THCa | CBDa | THC | CBD | A | B | THCa | THC | CBDa | CBD |
| 18 | 24.2 | 0.1 | 67 | 1 | 1 | 0.35 | 18 | 17 | 0.1 | 0.3 |
| 19 | 1 | 16 | 4 | 64 | 1 | 0.35 | 1 | 1 | 12 | 17 |
| 20 | 24.2 | 0.1 | 30 | 34 | 1 | 0.25 | 19 | 6 | 0.1 | 7 |
| 21 | 7.8 | 13.3 | 4 | 64 | 1 | 0.3 | 6 | 1 | 10 | 15 |
| 22 | 7.8 | 13.3 | 30 | 34 | 1 | 0.3 | 6 | 7 | 10 | 8 |
| 23 | 7.8 | 13.3 | 67 | 1 | 1 | 0.3 | 6 | 15 | 10 | 0.2 |
| 24 | 14.8 | 10.2 | 30 | 34 | 1 | 0.3 | 11 | 7 | 8 | 8 |

Example 25: Production of a cannabis tablet.
10gr of blends prepared according to Examples 2-23 were introduced into a tablet-making machine. 50 tablets were formed. The tablets sink when put in 95wt% ethanol solution and float when put in water.

### Examples 26 - 37

The methods of examples 2-23 were used to produce blends of compositions summarized in the following Table 4:

**Table 4**

| | Cannabinoids concentration (%) | | | |
|---|---|---|---|---|
| Example # | THCa | THC | CBDa | CBD |
| 26 | 3 | 1 | 1 | 5 |
| 27 | 1 | 2 | 8 | 3 |
| 28 | 5 | 0.5 | 6 | 10 |
| 29 | 3 | 3 | 12 | 7 |
| 30 | 0.5 | 5 | 11 | 9 |
| 31 | 5 | 0.5 | 16 | 8 |
| 32 | 8 | 3 | 9 | 2 |
| 33 | 7 | 4 | 15 | 7 |
| 34 | 16 | 4 | 12 | 3 |
| 38 | 22 | 6 | 5 | 2 |
| 36 | 24 | 8 | 4 | 1 |
| 37 | 25 | 9 | 5 | 2 |

Thus, the scope of the invention shall include all modifications and variations that may fall within the scope of the attached claims. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A homogeneous cannabis plant material composition comprising tetrahydrocannabinol (THC) and cannabidiol (CBD), wherein at least 70wt% of the cannabis plant material is cannabis flower, wherein the plant material is in the form of comminuted particles, and wherein
(i) tetrahydrocannabinol and cannabidiol concentrations are selected from the group consisting of
(a) 0.01-6wt% tetrahydrocannabinol and 18-24wt% cannabidiol;
(b) 0.01-6wt% tetrahydrocannabinol and 24-28wt% cannabidiol;
(c) 0.01-6wt% tetrahydrocannabinol and more than 28wt% cannabidiol;
(d) 6-12wt% tetrahydrocannabinol and 12-18wt% cannabidiol;
(e) 6-12wt % tetrahydrocannabinol and 18-24wt% cannabidiol;
(f) 6-12wt % tetrahydrocannabinol and more than 24wt% cannabidiol;
(g) 12-18wt% tetrahydrocannabinol and 6-12wt% cannabidiol;
(h) 12-18wt% tetrahydrocannabinol and 12-18wt% cannabidiol;
(i) 12-18wt% tetrahydrocannabinol and 18-24wt% cannabidiol;
(j) 12-18wt % tetrahydrocannabinol and more than 24wt% cannabidiol;
(k) 18-24wt% tetrahydrocannabinol and 6-12wt% cannabidiol;
(l) 18-24wt% tetrahydrocannabinol and 12-18wt% cannabidiol;
(m) 18-24wt% tetrahydrocannabinol and 18-24wt% cannabidiol;
(n) 18-24wt % tetrahydrocannabinol and more than 24wt% cannabidiol;
(o) 24-30wt% tetrahydrocannabinol and 6-12wt% cannabidiol;
(p) 24-30wt% tetrahydrocannabinol and 12-18wt% cannabidiol;
(q) 30-36wt% tetrahydrocannabinol and 0.01-6wt% cannabidiol;
(r) 30-36wt% tetrahydrocannabinol and 6-12wt% cannabidiol;
(s) 30-36wt% tetrahydrocannabinol and 12-18wt% cannabidiol;
(t) more than 36wt% tetrahydrocannabinol and 0.01-3wt% cannabidiol;
(u) more than 36wt% tetrahydrocannabinol and 3-6wt% cannabidiol; or
(v) more than 36wt% tetrahydrocannabinol and more than 6wt% cannabidiol; and wherein
(ii) at least 70 wt% of the comminuted cannabis plant material is of a size greater than 0.3 millimeter and less than 4.2millimeters;
(iii) the composition comprises at least 5wt% moisture and less than 20wt% moisture;
(iv) the homogeneous composition, when separated into at least a 10 gram sample, contains a tetrahydrocannabinol concentration in a 1 gram fraction of the at least 10 gram sample within 15% of the tetrahydrocannabinol concentration in a separate 1gram fraction of said at least 10 gram sample; and
(v) the homogeneous composition, when separated into at least a 10 gram sample, contains a cannabidiol concentration in a 1 gram fraction of the at least 10 gram sample within 15% of the cannabidiol concentration in a separate 1gram fraction of said at least 10 gram sample.

2. The homogeneous composition of Claim 1, wherein the tetrahydrocannabinol comprises tetrahydrocannabinol and tetrahydrocannabinolic acid and wherein the weight per weight ratio between tetrahydrocannabinol and tetrahydrocannabinolic acid is in the range between 20 to 1 and 1 to 20 and wherein the cannabidiol comprises cannabidiol and cannabidiolic acid and wherein the weight per weight ratio between cannabidiol and cannabidiolic acid is in the range between 20 to 1 and 1 to 20.

3. The homogeneous composition of Claim 1, having a bulk density greater than 0.02 gram/milliliter and less than 0.6 gram/milliliter.

4. A product comprising the homogeneous composition of Claim 1, wherein the product is selected from the group consisting of a cigarette and a vaporizer cartridge.

5. A method for producing the homogeneous comminuted cannabis plant material composition of Claim 1 comprising
(i) providing a tetrahydrocannabinol-comprising cannabis plant material;
(ii) providing a cannabidiol-comprising cannabis plant material;
(a) comminuting said tetrahydrocannabinol-comprising cannabis plant material, and said cannabidiol-comprising cannabis plant material to provide two separate comminuted homogeneous cannabis plant materials wherein at least 70 wt% of each comminuted cannabis plant material is of a size greater than 0.3 millimeter and less than 4.2millimeters; and/or (b) comminuting one of said tetrahydrocannabinol-comprising cannabis plant materials, and said cannabidiol-comprising cannabis plant material to provide comminuted cannabis plant material wherein at least 70 wt% of the comminuted cannabis plant material is of a size greater than 0.3 millimeter and less than 4.2millimeters, whereby a comminuted homogeneous cannabis plant material and a non-comminuted cannabis plant material are obtained and extracting the non-comminuted cannabis plant material to form a cannabinoid-comprising extract; and
(iii) (a) combining said two separate comminuted homogeneous cannabis plant materials of step (ii)(a) in a proportion calculated to provide said tetrahydrocannabinol and cannabidiol concentrations of said composition, and/or (b) combining said comminuted homogeneous cannabis plant material and said cannabinoid-comprising extract of step (ii)(b) in a proportion to provide said tetrahydrocannabinol and cannabidiol concentrations of said homogeneous comminuted cannabis plant material composition.

6. The method of Claim 5, wherein said extracting comprises
(i) optionally comminuting the non-comminuted cannabis plant material of step (ii)(b), whereby comminuted cannabis plant material is formed;
(ii) contacting said non-comminuted cannabis plant material or said comminuted cannabis plant material with a liquid extractant for at least 1 minute to form cannabinoid-depleted plant material and a cannabinoid-comprising extractant, and
(iii) separating said cannabinoid-depleted plant material from said cannabinoid-comprising extractant to form separated cannabinoid-comprising extractant.

7. The method of Claim 5, further comprising comminuting said two comminuted cannabis plant materials or said comminuted cannabis plant material and said cannabinoid-comprising extract with said cannabinoid-depleted plant material in a proportion calculated to form the said composition.

8. The method of Claim 5, comprising blending said comminuted cannabis plant material and said cannabinoid-comprising extract with said cannabinoid-depleted plant material in a proportion calculated to form the said composition.

9. The method of Claim 5, further comprising adding said homogeneous composition to a cigarettes tube or a cigarette paper, whereby a cigarette is formed.

10. A cannabis cigarette produced according to the method of Claim 9, wherein said cigarette contains a top fraction and a bottom fraction and the concentration of tetrahydrocannabinol in a 0.1 gram sample from the top fraction of the cigarette is within 15% of tetrahydrocannabinol concentration in a 0.1 gram sample from the bottom fraction of the cigarette and wherein cannabidiol in a 0.1 gram sample from the top fraction of the cigarette is within 15% of cannabidiol concentration in a 0.1 gram sample from the bottom fraction of the cigarette.

11. A batch of cannabis cigarettes produced according to the method of Claim 9, wherein the content of tetrahydrocannabinol in one third of the batch of the cigarettes is within 15% of the tetrahydrocannabinol content in a separate third of the batch of the cigarettes and wherein the content of cannabidiol in one third of the batch of the cigarettes is within 15% of the cannabidiol content in a separate third of the batch of the cigarettes.

12. The method of Claim 5, further comprising pressing said homogeneous comminuted composition to form a homogeneous cannabis tablet.

13. A homogeneous cannabis tablet produced according to the method of Claim 5, wherein said tablet has a bulk density of at least 0.1 gram/milliliter and less than 1.2 gram/milliliter.

## Patentansprüche

1. Homogene Cannabispflanzenmaterialzusammensetzung, die Tetrahydrocannabinol (THC) und Cannabidiol (CBD) umfasst, wobei zumindest 70 Gew.-% des Cannabispflanzenmaterials Cannabisblüten sind, wobei das Pflanzenmaterial in der Form von zerkleinerten Partikeln ist, und wobei
(i) Tetrahydrocannabinol- und Cannabidiolkonzentrationen ausgewählt sind aus der Gruppe bestehend aus
(a) 0,01-6 Gew.-% Tetrahydrocannabinol und 18-24 Gew.-% Cannabidiol;
(b) 0,01-6 Gew.-% Tetrahydrocannabinol und 24-28 Gew.-% Cannabidiol;
(c) 0,01-6 Gew.-% Tetrahydrocannabinol und mehr als 28 Gew.-% Cannabidiol;
(d) 6-12 Gew.-% Tetrahydrocannabinol und 12-18 Gew.-% Cannabidiol;
(e) 6-12 Gew.-% Tetrahydrocannabinol und 18-24 Gew.-% Cannabidiol;
(f) 6-12 Gew.-% Tetrahydrocannabinol und mehr als 24 Gew.-% Cannabidiol;
(g) 12-18 Gew.-% Tetrahydrocannabinol und 6-12 Gew.-% Cannabidiol;
(h) 12-18 Gew.-% Tetrahydrocannabinol und 12-18 Gew.-% Cannabidiol;
(i) 12-18 Gew.-% Tetrahydrocannabinol und 18-24 Gew.-% Cannabidiol;
(j) 12-18 Gew.-% Tetrahydrocannabinol und mehr als 24 Gew.-% Cannabidiol;
(k) 18-24 Gew.-% Tetrahydrocannabinol und 6-12 Gew.-% Cannabidiol;
(l) 18-24 Gew.-% Tetrahydrocannabinol und 12-18 Gew.-% Cannabidiol;
(m) 18-24 Gew.-% Tetrahydrocannabinol und 18-24 Gew.-% Cannabidiol;
(n) 18-24 Gew.-% Tetrahydrocannabinol und mehr als 24 Gew.-% Cannabidiol;
(o) 24-30 Gew.-% Tetrahydrocannabinol und 6-12 Gew.-% Cannabidiol;
(p) 24-30 Gew.-% Tetrahydrocannabinol und 12-18 Gew.-% Cannabidiol;
(q) 30-36 Gew.-% Tetrahydrocannabinol und 0,01-6 Gew.-% Cannabidiol;
(r) 30-36 Gew.-% Tetrahydrocannabinol und 6-12 Gew.-% Cannabidiol;
(s) 30-36 Gew.-% Tetrahydrocannabinol und 12-18 Gew.-% Cannabidiol;
(t) mehr als 36 Gew.-% Tetrahydrocannabinol und 0,01-3 Gew.-% Cannabidiol;
(u) mehr als 36 Gew.-% Tetrahydrocannabinol und 3-6 Gew.-% Cannabidiol; oder
(v) mehr als 36 Gew.-% Tetrahydrocannabinol und mehr als 6 Gew.-% Cannabidiol; und wobei
(ii) zumindest 70 Gew.-% des zerkleinerten Cannabispflanzenmaterials von einer Größe größer als 0,3 Millimeter und weniger als 4,2 Millimeter sind;
(iii) die Zusammensetzung zumindest 5 Gew.-% Feuchtigkeit und weniger als 20 Gew.-% Feuchtigkeit umfasst;
(iv) die homogene Zusammensetzung, wenn in zumindest eine 10 Gramm große Probe aufgeteilt, eine Tetrahydrocannabinolkonzentration in einem 1 Gramm großen Anteil der zumindest 10 Gramm großen Probe innerhalb von 15 % der Tetrahydrocannabinolkonzentration in einem separaten 1 Gramm großen Anteil der zumindest 10 Gramm großen Probe enthält; und
(v) die homogene Zusammensetzung, wenn in zumindest eine 10 Gramm große Probe aufgeteilt, eine Cannabidiolkonzentration in einem 1 Gramm großen Anteil der zumindest einen 10 Gramm großen Probe innerhalb von 15 % der Cannabidiolkonzentration in einem separaten 1 Gramm großen Anteil der zumindest einen 10 Gramm großen Probe enthält.

2. Homogene Zusammensetzung nach Anspruch 1, wobei das Tetrahydrocannabinol Tetrahydrocannabinol und Tetrahydrocannabinolsäure umfasst und wobei das Gewichtsverhältnis zwischen Tetrahydrocannabinol und Tetrahydrocannabinolsäure in dem Bereich zwischen 20 zu 1 und 1 zu 20 ist und wobei das Cannabidiol Cannabidiol und Cannabidiolsäure umfasst und wobei das Gewichtsverhältnis zwischen Cannabidiol und Cannabidiolsäure in dem Bereich zwischen 20 zu 1 und 1 zu 20 ist.

3. Homogene Zusammensetzung nach Anspruch 1 mit einer Schüttdichte von mehr als 0,02 Gramm/Milliliter und weniger als 0,6 Gramm/Milliliter.

4. Produkt, das die homogene Zusammensetzung nach Anspruch 1 umfasst, wobei das Produkt aus der Gruppe ausgewählt ist, die aus einer Zigarette und einer Verdampferkartusche besteht.

5. Verfahren zur Herstellung der homogenen zerkleinerten Cannabispflanzenmaterialzusammensetzung nach Anspruch 1, umfassend
(i) Bereitstellen eines Tetrahydrocannabinol umfassenden Cannabispflanzenmaterials;
(ii) Bereitstellen eines Cannabidiol umfassenden Cannabispflanzenmaterials;
(a) Zerkleinern des Tetrahydrocannabinol umfassenden Cannabispflanzenmaterials und des Cannabidiol umfassenden Cannabispflanzenmaterials, um zwei separate zerkleinerte homogene Cannabispflanzenmaterialien bereitzustellen, wobei zumindest 70 Gew.-% jedes zerkleinerten Cannabispflanzenmaterials von einer Größe größer als 0,3 Millimeter und weniger als 4,2 Millimeter sind; und/oder (b) Zerkleinern von einem der Tetrahydrocannabinol umfassenden Cannabispflanzenmaterialien und des Cannabidiol umfassenden Cannabispflanzenmaterials, um zerkleinertes Cannabispflanzenmaterial bereitzustellen, wobei zumindest 70 Gew.-% des zerkleinerten Cannabispflanzenmaterials von einer Größe größer als 0,3 Millimeter und weniger als 4,2 Millimeter sind, wodurch ein zerkleinertes homogenes Cannabispflanzenmaterial und ein nicht zerkleinertes Cannabispflanzenmaterial erhalten werden, und Extrahieren des nicht zerkleinerten Cannabispflanzenmaterials, um einen Cannabinoid umfassenden Extrakt zu bilden; und
(iii) (a) Kombinieren der zwei separaten zerkleinerten homogenen Cannabispflanzenmaterialien aus Schritt (ii)(a) in einem Verhältnis, das berechnet ist, um die Tetrahydrocannabinol- und Cannabidiolkonzentrationen der Zusammensetzung bereitzustellen, und/oder
(b) Kombinieren des zerkleinerten homogenen Cannabispflanzenmaterials und des Cannabinoid umfassenden Extrakts aus Schritt (ii)(b) in einem Verhältnis, um die Tetrahydrocannabinol- und Cannabidiolkonzentrationen der homogenen zerkleinerten Cannabispflanzenmaterialzusammensetzung bereitzustellen.

6. Verfahren nach Anspruch 5, wobei das Extrahieren Folgendes umfasst
(i) optional Zerkleinern des nicht zerkleinerten Cannabispflanzenmaterials aus Schritt (ii)(b), wodurch zerkleinertes Cannabispflanzenmaterial gebildet wird;
(ii) Kontaktieren des nicht zerkleinerten Cannabispflanzenmaterials oder des zerkleinerten Cannabispflanzenmaterials mit einem flüssigen Extraktionsmittel für zumindest 1 Minute, um cannabinoidarmes Pflanzenmaterial und ein Cannabinoid umfassendes Extraktionsmittel zu bilden, und
(iii) Trennen des cannabinoidarmen Pflanzenmaterials von dem Cannabinoid umfassenden Extraktionsmittel, um getrenntes Cannabinoid umfassendes Extraktionsmittel zu bilden.

7. Verfahren nach Anspruch 5, ferner umfassend Zerkleinern der zwei zerkleinerten Cannabispflanzenmaterialien oder des zerkleinerten Cannabispflanzenmaterials und des Cannabinoid umfassenden Extrakts mit dem cannabinoidarmen Pflanzenmaterial in einem Verhältnis, das berechnet ist, um die Zusammensetzung zu bilden.

8. Verfahren nach Anspruch 5, umfassend Mischen des zerkleinerten Cannabispflanzenmaterials und des Cannabinoid umfassenden Extrakts mit dem cannabinoidarmen Pflanzenmaterial in einem Verhältnis, das berechnet ist, um die Zusammensetzung zu bilden.

9. Verfahren nach Anspruch 5, ferner umfassend Hinzufügen der homogenen Zusammensetzung zu einer Zigarettenhülse oder einem Zigarettenpapier, wodurch eine Zigarette gebildet wird.

10. Cannabiszigarette, die nach dem Verfahren von Anspruch 9 hergestellt ist, wobei die Zigarette eine obere Fraktion und eine untere Fraktion enthält und die Konzentration von Tetrahydrocannabinol in einer 0,1 Gramm großen Probe aus der oberen Fraktion der Zigarette innerhalb von 15 % der Tetrahydrocannabinolkonzentration in einer 0,1 Gramm großen Probe aus der unteren Fraktion der Zigarette ist und wobei Cannabidiol in einer 0,1 Gramm großen Probe aus der oberen Fraktion der Zigarette innerhalb von 15 % der Cannabidiolkonzentration in einer 0,1 Gramm großen Probe aus der unteren Fraktion der Zigarette ist.

11. Charge von Cannabiszigaretten, die nach dem Verfahren von Anspruch 9 hergestellt ist, wobei der Gehalt an Tetrahydrocannabinol in einem Drittel der Charge der Zigaretten innerhalb von 15 % des Tetrahydrocannabinolgehalts in einem separaten Drittel der Charge der Zigaretten ist und wobei der Gehalt an Cannabidiol in einem Drittel der Charge der Zigaretten innerhalb von 15 % des Cannabidiolgehalts in einem separaten Drittel der Charge der Zigaretten ist.

12. Verfahren nach Anspruch 5, ferner umfassend Pressen der homogenen zerkleinerten Zusammensetzung, um eine homogene Cannabistablette zu bilden.

13. Homogene Cannabistablette, die nach dem Verfahren von Anspruch 5 hergestellt ist, wobei die Tablette eine Schüttdichte von zumindest 0,1 Gramm/Milliliter und weniger als 1,2 Gramm/Milliliter aufweist.

## Revendications

1. Composition homogène de matière végétale de cannabis comprenant du tétrahydrocannabinol (THC) et du cannabidiol (CBD), dans laquelle au moins 70 % en poids de la matière végétale de cannabis est constituée de fleur de cannabis, dans laquelle la matière végétale se présente sous la forme de particules broyées, et dans laquelle
(i) les concentrations de tétrahydrocannabinol et de cannabidiol sont choisies dans le groupe constitué de
(a) 0,01 à 6 % en poids de tétrahydrocannabinol et 18 à 24 % en poids de cannabidiol ;
(b) 0,01 à 6 % en poids de tétrahydrocannabinol et 24 à 28 % en poids de cannabidiol ;
(c) 0,01 à 6 % en poids de tétrahydrocannabinol et plus de 28 % en poids de cannabidiol ;
(d) 6 à 12 % en poids de tétrahydrocannabinol et 12 à 18 % en poids de cannabidiol ;
(e) 6 à 12 % en poids de tétrahydrocannabinol et 18 à 24 % en poids de cannabidiol ;
(f) 6 à 12 % en poids de tétrahydrocannabinol et plus de 24 % en poids de cannabidiol ;
(g) 12 à 18 % en poids de tétrahydrocannabinol et 6 à 12 % en poids de cannabidiol ;
(h) 12 à 18 % en poids de tétrahydrocannabinol et 12 à 18 % en poids de cannabidiol ;
(i) 12 à 18 % en poids de tétrahydrocannabinol et 18 à 24 % en poids de cannabidiol ;
(j) 12 à 18 % en poids de tétrahydrocannabinol et plus de 24 % en poids de cannabidiol ;
(k) 18 à 24 % en poids de tétrahydrocannabinol et 6 à 12 % en poids de cannabidiol ;
(l) 18 à 24 % en poids de tétrahydrocannabinol et 12 à 18 % en poids de cannabidiol ;
(m) 18 à 24 % en poids de tétrahydrocannabinol et 18 à 24 % en poids de cannabidiol ;
(n) 18 à 24 % en poids de tétrahydrocannabinol et plus de 24 % en poids de cannabidiol ;
(o) 24 à 30 % en poids de tétrahydrocannabinol et 6 à 12 % en poids de cannabidiol ;
(p) 24 à 30 % en poids de tétrahydrocannabinol et 12 à 18 % en poids de cannabidiol ;
(q) 30 à 36 % en poids de tétrahydrocannabinol et 0,01 à 6 % en poids de cannabidiol ;
(r) 30 à 36 % en poids de tétrahydrocannabinol et 6 à 12 % en poids de cannabidiol ;
(s) 30 à 36 % en poids de tétrahydrocannabinol et 12 à 18 % en poids de cannabidiol ;
(t) plus de 36 % en poids de tétrahydrocannabinol et 0,01 à 3 % en poids de cannabidiol ;
(u) plus de 36 % en poids de tétrahydrocannabinol et 3 à 6 % en poids de cannabidiol ; ou
(v) plus de 36 % en poids de tétrahydrocannabinol et plus de 6 % en poids de cannabidiol ; et dans laquelle
(ii) au moins 70 % en poids de la matière végétale de cannabis broyée est d'une taille supérieure à 0,3 millimètre et inférieure à 4,2 millimètres ;
(iii) la composition comprend au moins 5 % en poids d'humidité et moins de 20 % en poids d'humidité ;
(iv) la composition homogène, lorsqu'elle est séparée en un échantillon d'au moins 10 grammes, contient une concentration de tétrahydrocannabinol dans une fraction de 1 gramme de l'échantillon d'au moins 10 grammes dans les 15 % de la concentration de tétrahydrocannabinol dans une fraction séparée de 1 gramme dudit échantillon d'au moins 10 grammes ; et
(v) la composition homogène, lorsqu'elle est séparée en un échantillon d'au moins 10 grammes, contient une concentration de cannabidiol dans une fraction de 1 gramme de l'échantillon d'au moins 10 grammes dans les 15 % de la concentration de cannabidiol dans une fraction séparée de 1 gramme dudit échantillon d'au moins 10 grammes.

2. Composition homogène selon la revendication 1, dans laquelle le tétrahydrocannabinol comprend du tétrahydrocannabinol et de l'acide tétrahydrocannabinolique et dans laquelle le rapport poids par poids entre le tétrahydrocannabinol et l'acide tétrahydrocannabinolique est compris dans la plage de 20 à 1 et de 1 à 20 et dans laquelle le cannabidiol comprend du cannabidiol et de l'acide cannabidiolique et dans laquelle le rapport poids par poids entre le cannabidiol et l'acide cannabidiolique est compris dans la plage de 20 à 1 et de 1 à 20.

3. Composition homogène selon la revendication 1, comportant une masse volumique apparente supérieure à 0,02 gramme/millilitre et inférieure à 0,6 gramme/millilitre.

4. Produit comprenant la composition homogène selon la revendication 1, dans lequel le produit est choisi dans le groupe constitué d'une cigarette et d'une cartouche de vaporisateur.

5. Procédé permettant la production de la composition homogène de matière végétale de cannabis broyée selon la revendication 1, comprenant
(i) la fourniture d'une matière végétale de cannabis comprenant du tétrahydrocannabinol ;
(ii) la fourniture d'une matière végétale de cannabis comprenant du cannabidiol ;
(a) le broyage de ladite matière végétale de cannabis comprenant du tétrahydrocannabinol et de ladite matière végétale de cannabis comprenant du cannabidiol pour fournir deux matières végétales de cannabis homogènes broyées, séparées, dans lequel au moins 70 % en poids de chaque matière végétale de cannabis broyée est d'une taille supérieure à 0,3 millimètre et inférieure à 4,2 millimètres ; et/ou (b) le broyage de l'une desdites matières végétales de cannabis comprenant du tétrahydrocannabinol et de ladite matière végétale de cannabis comprenant du cannabidiol pour fournir une matière végétale de cannabis broyée, dans lequel au moins 70 % en poids de la matière végétale de cannabis broyée est d'une taille supérieure à 0,3 millimètre et inférieure à 4,2 millimètres, moyennant quoi une matière végétale de cannabis homogène broyée et une matière végétale de cannabis non broyée sont obtenues et l'extraction de la matière végétale de cannabis non broyée pour former un extrait comprenant des cannabinoïdes ; et
(iii) (a) la combinaison desdits deux matières végétales de cannabis homogènes broyées, séparées, de l'étape (ii)(a) dans une proportion calculée pour fournir lesdites concentrations de tétrahydrocannabinol et de cannabidiol de ladite composition, et/ou
(b) la combinaison de ladite matière végétale de cannabis homogène broyée et dudit extrait comprenant des cannabinoïdes de l'étape (ii)(b) dans une proportion pour fournir lesdites concentrations de tétrahydrocannabinol et de cannabidiol de ladite composition de matière végétale de cannabis homogène broyée.

6. Procédé selon la revendications 5, dans lequel ladite extraction comprend
(i) éventuellement le broyage de la matière végétale de cannabis non broyée de l'étape (ii)(b), moyennant quoi la matière végétale de cannabis broyée est formée ;
(ii) la mise en contact de ladite matière végétale de cannabis non broyée ou de ladite matière végétale de cannabis broyée avec un extractant liquide pendant au moins 1 minute pour former une matière végétale appauvrie en cannabinoïdes et un extractant comprenant des cannabinoïdes, et
(iii) la séparation de ladite matière végétale appauvrie en cannabinoïdes dudit extractant comprenant des cannabinoïdes pour former un extractant comprenant des cannabinoïdes séparé.

7. Procédé selon la revendication 5, comprenant en outre le broyage desdites deux matières végétales de cannabis broyées ou de ladite matière végétale de cannabis broyée et dudit extrait comprenant des cannabinoïdes avec ladite matière végétale appauvrie en cannabinoïdes dans une proportion calculée pour former ladite composition.

8. Procédé selon la revendication 5, comprenant le mélange de ladite matière végétale de cannabis broyée et dudit extrait comprenant des cannabinoïdes avec ladite matière végétale appauvrie en cannabinoïdes dans une proportion calculée pour former ladite composition.

9. Procédé selon la revendication 5, comprenant en outre l'ajout de ladite composition homogène à un tube à cigarettes ou à un papier à cigarettes, moyennant quoi une cigarette est formée.

10. Cigarette de cannabis produite selon le procédé de la revendication 9, dans laquelle ladite cigarette contient une fraction haute et une fraction basse et la concentration de tétrahydrocannabinol dans un échantillon de 0,1 gramme de la fraction haute de la cigarette se situe dans les 15 % de la concentration de tétrahydrocannabinol dans un échantillon de 0,1 gramme de la fraction basse de la cigarette et dans laquelle le cannabidiol dans un échantillon de 0,1 gramme de la fraction haute de la cigarette se situe dans les 15 % de la concentration de cannabidiol dans un échantillon de 0,1 gramme de la fraction basse de la cigarette.

11. Lot de cigarettes de cannabis produites selon le procédé de la revendication 9, dans lequel la teneur en tétrahydrocannabinol dans un tiers du lot de cigarettes se situe dans les 15 % de la teneur en tétrahydrocannabinol dans un tiers distinct du lot de cigarettes et dans lequel la teneur en cannabidiol dans un tiers du lot de cigarettes se situe dans les 15 % de la teneur en cannabidiol dans un tiers distinct du lot de cigarettes.

12. Procédé selon la revendication 5, comprenant en outre le pressage de ladite composition broyée homogène pour former un comprimé de cannabis homogène.

13. Comprimé de cannabis homogène produit selon le procédé de la revendication 5, dans lequel ledit comprimé comporte une masse volumique apparente d'au moins 0,1 gramme/millilitre et inférieure à 1,2 gramme/millilitre.
